(19) 

Europäisches
Patentamt
European
Patent Office
Office européen
des brevets

(11) **EP 2 890 712 B1**

(12) **EUROPEAN PATENT SPECIFICATION**

(45) Date of publication and mention
of the grant of the patent:
**01.05.2019 Bulletin 2019/18**

(21) Application number: **13756076.9**

(22) Date of filing: **26.08.2013**

(51) Int Cl.:
*C07K 16/28* (2006.01)     *C07K 16/18* (2006.01)
*C07K 16/46* (2006.01)     *A61K 39/395* (2006.01)
*A61P 25/28* (2006.01)

(86) International application number:
**PCT/EP2013/067595**

(87) International publication number:
**WO 2014/033074 (06.03.2014 Gazette 2014/10)**

(54) **BLOOD BRAIN BARRIER SHUTTLE**

BLUT-HIRN-SCHRANKEN-SHUTTLE

NAVETTE DE LA BARRIÈRE HÉMATO-ENCÉPHALIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.08.2012 EP 12182181**

(43) Date of publication of application:
**08.07.2015 Bulletin 2015/28**

(60) Divisional application:
**17199410.6 / 3 315 514**

(73) Proprietor: **F. Hoffmann-La Roche AG
4070 Basel (CH)**

(72) Inventors:
• **BOHRMANN, Bernd
4125 Riehen (CH)**
• **FRESKGARD, Per-Ola
4153 Reinach BL (CH)**
• **MAIER, Peter
88400 Biberach an der Riss (DE)**
• **NIEWOEHNER, Jens
81476 München (DE)**
• **TISSOT-DAGUETTE, Alain
82061 Neuried (DE)**
• **URICH, Eduard
4053 Basel (CH)**

(74) Representative: **Küng, Peter
F. Hoffmann-La Roche AG
Patent Department
Grenzacherstrasse 124
4070 Basel (CH)**

(56) References cited:
**WO-A1-2007/068429     WO-A1-2012/075037
US-A1- 2005 147 613     US-A1- 2010 081 796
US-A1- 2010 256 338**

• **KONTERMANN ROLAND E: "Dual targeting
strategies with bispecific antibodies", MABS, vol.
4, no. 2, March 2012 (2012-03), pages 182-197,
XP002698995,**
• **NIEWOEHNER JENS ET AL: "Increased Brain
Penetration and Potency of a Therapeutic
Antibody Using a Monovalent Molecular Shuttle",
NEURON, CELL PRESS, US, vol. 81, no. 1, 8
January 2014 (2014-01-08), pages 49-60,
XP028813101, ISSN: 0896-6273, DOI:
10.1016/J.NEURON.2013.10.061**
• **BELL ROBERT D ET AL: "Breaching the
blood-brain barrier for drug delivery.", NEURON
08 JAN 2014, vol. 81, no. 1, 8 January 2014
(2014-01-08), pages 1-3, ISSN: 1097-4199**

## Description

The present invention relates to a blood brain barrier shuttle that binds receptors on the blood brain barrier (R/BBB) and methods of using the same.

BACKGROUND

Brain penetration of neurological disorder drugs such as e.g. large biotherapeutic drugs or small molecule drugs having a low brain penetration, is strictly limited by the extensive and impermeable blood-brain barrier (BBB) together with the other cell component in the neurovascular unit (NVU). Many strategies to overcome this obstacle have been tested and one is to utilize transcytosis pathways mediated by endogenous receptors expressed on the brain capillary endothelium. Recombinant proteins such as monoclonal antibodies or peptides have been designed against these receptors to enable receptor-mediated delivery of biotherapeutics to the brain. However, strategies to maximize brain uptake while minimizing miss-sorting within the brain endothelial cells (BECs), and the extent of accumulation within certain organelles (especially organelles that leads to degradation of the biotherapeutic) in BECs, remain unexplored.

Monoclonal antibodies and other biotherapeutics have huge therapeutic potential for treatment of pathology in the central nervous system (CNS). However, their route into the brain is prevented by BBB. Previous studies have illustrated that a very small percentage (approximately 0.1%) of an IgG injected in the bloodstream are able to penetrate into the CNS compartment (Felgenhauer, Klin. Wschr. 52: 1158-1164 (1974)). This will certainly limit any pharmacological effect due to the low concentration within CNS of the antibody.

WO 2012/075037 discloses a bispecific full length antibody which comprises an anti-BACE1 Fab fragment and an anti-transferrin receptor Fab fragment. This bispecific antibody functions as a blood brain barrier shuttle.

US 2005/147613 discloses bispecific antibodies comprising either an anti-transferrin receptor full length antibody and an anti-Abeta full length antibody or an anti-transferrin Fab fragment and an anti-abeta Fab fragment. Both constructs function as blood brain barrier shuttle.

Therefore, there is a need for delivery systems of neurological disorder drugs across the BBB to shuttle the drugs into the brain efficiently.

SUMMARY

In a first aspect, the present invention provides a blood brain barrier shuttle comprising a brain effector entity, a linker and one monovalent binding entity which binds to a transferrin receptor, wherein the linker couples the effector entity to the monovalent binding entity which binds to the transferrin receptor, wherein the monovalent binding entity which binds to the transferrin receptor comprises one scFab directed to the transferrin receptor recognizing an epitope in the transferrin receptor comprised within the amino acid sequence of Seq. Id. No. 14, 15 or 16 and wherein the brain effector entity is selected from the group consisting of neurological disorder drugs, neurotrophic factors and antibodies directed to a brain target selected from the group consisting of β-secretase 1, Aβ, epidermal growth factor, epidermal growth factor receptor 2, Tau, phosphorylated Tau, apolipoprotein E4, alpha synuclein, oligomeric fragments of alpha synuclein, CD20, huntingtin, prion protein, leucine rich repeat kinase 2, parkin, presenilin 2, gamma secretase, death receptor 6, amyloid precursor protein, p75 neurotrophin receptor and caspase 6.

In a particular embodiment of the blood brain barrier shuttle, the brain effector entity is selected from the group consisting of proteins, polypeptides and peptides.

In a particular embodiment of the blood brain barrier shuttle, the brain effector entity comprises a full length antibody directed to a brain target, preferably a full length IgG.

In a particular embodiment of the blood brain barrier shuttle, the blood brain barrier shuttle comprises a full length IgG antibody as brain effector entity, the linker and one scFab which binds the transferrin receptor recognizing an epitope in the transferrin receptor comprised within the amino acid sequence of Seq. Id. No. 14, 15 or 16, wherein the scFab is coupled by the linker to the C-terminal end of the Fc part of one of the heavy chains of the IgG antibody.

In a particular embodiment of the blood brain barrier shuttle, the effector entity is a full length antibody directed to Aβ.

In a particular embodiment of the blood brain barrier shuttle, the antibody directed to Aβ comprises (a) H-CDR1 comprising the amino acid sequence of Seq. Id. No. 5, (b) H-CDR2 comprising the amino acid sequence of Seq. Id. No. 6, (c) H-CDR3 comprising the amino acid sequence of Seq. Id. No. 7, (d) L-CDR1 comprising the amino acid sequence of Seq. Id. No. 8, (e) L-CDR2 comprising the amino acid sequence of Seq. Id. No. 9 and (f) L-CDR3 comprising the amino acid sequence of Seq. Id. No. 10.

In a particular embodiment of the blood brain barrier shuttle, the antibody directed to Abeta comprises a $V_H$ domain comprising the amino acid sequence of Seq. Id. No. 11 and a $V_L$ domain comprising the amino acid sequence of Seq. Id. No. 12.

In a particular embodiment of the blood brain barrier shuttle, the first heavy chain of the antibody of the blood brain barrier shuttle directed to a brain target comprises a first dimerization module and the second heavy chain of the antibody of the blood brain barrier shuttle to a brain target comprises a second dimerization module allowing heterodimerization of the two heavy chains.

In a particular embodiment of the blood brain barrier shuttle, the first dimerization module of the first heavy chain of the antibody of the blood brain barrier shuttle directed to the brain target comprises knobs and the dimerization module of the second heavy chain of the antibody of the blood brain barrier shuttle directed to the brain target comprises holes according to the knobs into holes strategy.

In a particular embodiment of the blood brain barrier shuttle, the effector entity is a full length antibody directed to phosphorylated Tau.

In a particular embodiment of the blood brain barrier shuttle, the effector entity is a full length antibody directed to alpha synuclein.

In a particular embodiment of the blood brain barrier shuttle, the linker is a peptide linker, preferably a peptide which is an amino acid sequence with a length of at least 25 amino acids, more preferably with a length of 30 to 50 amino acids, in particular said linker is $(G_4S)_6G_2$ or $(G_4S)_4$.

The following three embodiments of the invention relate to a blood brain barrier shuttle wherein the brain effector entity is a protein, polypeptide or peptide with the proviso that the brain effector entity is not a full length antibody, in particular a full length IgG.

In a particular embodiment of the blood brain barrier shuttle, the monovalent binding entity which binds to the transferrin receptor comprises a CH2-CH3 Ig entity and one scFab which binds to the transferrin receptor recognizing an epitope in the transferrin receptor comprised within the amino acid sequence of Seq. Id. No. 14, 15 or 16, wherein the scFab is coupled to a C-terminal end of the CH2-CH3 Ig entity by a second linker.

In a particular embodiment of the blood brain barrier shuttle, the blood brain barrier shuttle comprises the brain effector entity, the linker, the CH2-CH3 Ig domain, the second linker and one scFab which binds to the transferrin receptor recognizing an epitope in the transferrin receptor comprised within the amino acid sequence of Seq. Id. No. 14, 15 or 16 , wherein the brain effector entity is coupled by the first linker to a N-terminal end of the CH2-CH3 Ig domain and the scFab is coupled to a C-terminal end of the CH2-CH3 Ig domain by the second linker.

In a particular embodiment of the blood brain barrier shuttle, the CH2-CH3 Ig entity is a CH2-CH3 IgG entity.

The blood brain barrier shuttle of the present invention can be used as a medicament, in particular it can be used for the treatment of a neurological disorder such as e.g. Alzheimer's disease.

The blood brain barrier shuttle of the present invention can be used to transport the brain effector entity across the blood brain barrier.

In a particular embodiment, the heavy chain of the IgG antibody of the blood brain barrier shuttle of the present invention coupled at its C-terminal end of the Fc part to the scFab as monovalent binding entity which binds to the blood brain barrier receptor has the following structure:

- IgG heavy chain,

- Linker coupling the C-terminal end of the Fc part of the IgG heavy chain to the N-terminal end of the VL domain of the scFab,

- Variable light chain domain (VL) and C-kappa light chain domain of the scFab,

- Linker coupling the C-terminal end of the C-kappa light chain domain of the scFab to the N-terminal end of the VH domain of the scFab,

- Variable heavy chain domain (VH) of the scFab antibody and IgG CH3 heavy chain domain.

In a second aspect the present invention provides a fusion protein to transport a brain effector entity across the blood brain barrier comprising a CH2-CH3 Ig entity, a linker and one scFab directed to a transferrin receptor recognizing an epitope in the transferrin receptor comprised within the amino acid sequence of Seq. Id. No. 14, 15 or 16, wherein the scFab is coupled to a C-terminal end of the CH2-CH3 Ig entity by the linker and wherein the brain effector entity is selected from the group consisting of neurological disorder drugs, neurotrophic factors and antibody fragments directed to a brain target selected from the group consisting of scFv, Fv, scFab, Fab, VHH, $F(ab')_2$ or peptides directed to a brain target and wherein the brain target is selected from the group consisting of β-secretase 1, Aβ, epidermal growth factor, epidermal growth factor receptor 2, Tau, phosphorylated Tau, apolipoprotein E4, alpha synuclein, oligomeric fragments of alpha synuclein, CD20, huntingtin, prion protein, leucine rich repeat kinase 2, parkin, presenilin 2, gamma secretase, death receptor 6, amyloid precursor protein, p75 neurotrophin receptor and caspase 6.

In a particular embodiment of the fusion protein of the present invention, the fusion protein of the present invention further comprises a linker at the N-terminal end of the CH2-CH3 Ig entity to couple the brain effector entity to the N-terminal end of the CH2-CH3 Ig entity.

In a particular embodiment of the fusion protein of the present invention, the linker is a peptide linker, in particular a peptide which is an amino acid sequence with a length of at least 15 amino acids, more particularly with a length of 20 to 50 amino acids, most particularly said linker has the amino acid sequence $(G_4S)_6G_2$ (Seq. Id. No. 13) or $(G_4S)_4$ (Seq. Id. No. 17).

In a particular embodiment of the fusion protein of the present invention, the CH2-CH3 Ig entity is a CH2-CH3 IgG entity.

In a third aspect the present invention provides a conjugate comprising a fusion protein of the present invention and a brain effector entity coupled to a N-terminal end of the CH2-CH3 Ig entity of the fusion protein of the present invention by a linker.

In a particular embodiment of the conjugate of the present invention, the brain effector entity is a neurotrophic factor and wherein the linker coupling the neurotrophic factor to the N-terminal end of the CH2-CH3 Ig entity is a peptide linker.

Furthermore, the present invention provides a pharmaceutical formulation comprising the conjugate of the present invention and a pharmaceutical carrier, the use of the conjugate as a medicament, in particular the use of the conjugate for the treatment of a neurodegenerative disorder, in particular Alzheimer's disease.

BRIEF DESCRIPTION OF THE FIGURES

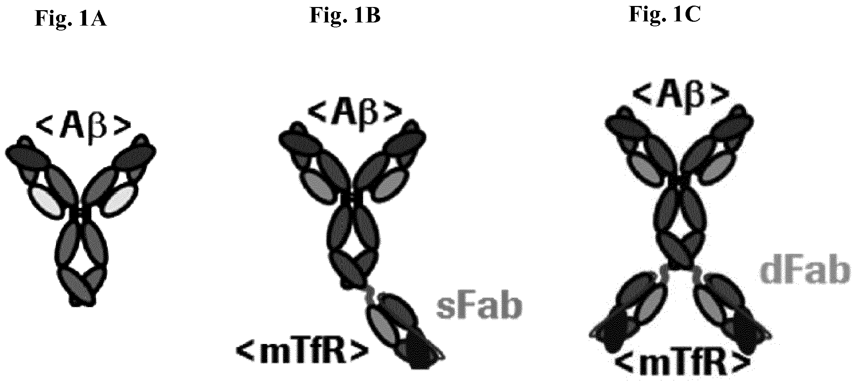

Fig. 1: Different format of blood brain barrier shuttles (fusion proteins) used in the examples. 1A: IgG directed to Aβ (mAb31). 1B: single Fab (sFab = single scFab) directed to TfR coupled to the Fc part of an IgG directed to Aβ (mAb31). 1C: double Fab (dFab = double scFab) directed to TfR coupled to the Fc part of an IgG directed to Aβ (mAb31). The scFab structure is fused to the C-terminal end of the heavy chain of the IgG antibody.

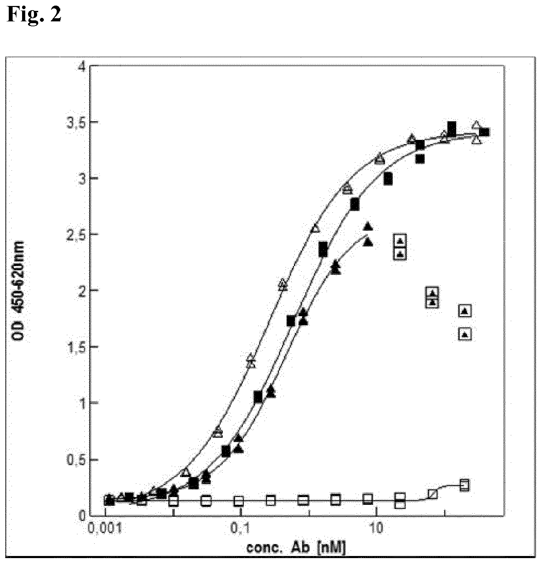

Fig. 2: Binding properties of the fusion proteins towards Aβ structures. The binding affinity was measured using an ELISA setup which shows that the Fab constructs have preserved Aβ binding properties. Binding of mAb31-8D3 constructs to Abeta fibrils. While 8D3 (open squares) does not bind to immobilized Abeta fibrils, mAb31-8D3-dFab (filled squares), mAb31-8D3- sFab (open triangles) and mAb31 (filled triangles) bind with comparable affinities.

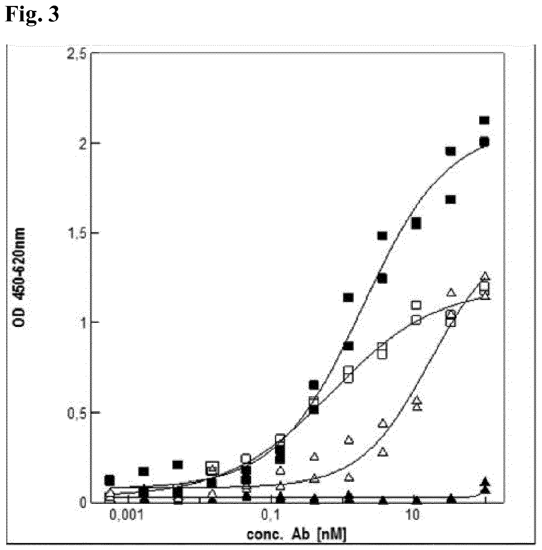

Fig. 3: Binding properties of the constructs towards the Transferrin receptor (TfR). The binding affinity was measure using an ELISA setup which shows that only the Fab constructs binds the Transferrin receptor (TfR) and the double Fab construct have slightly higher apparent affinity due to the bivalent binding mode. Binding of mAb31-8D3 constructs to mTfR. While mAb31 (filled triangles) does not bind to immobilized mTfR, mAb31-8D3-dFab (filled squares) binds with an affinity comparable to that of the 8D3 parent antibody (open squares). The monovalent construct mAb31-8D3- sFab (open triangles) shows an intermediate binding affinity.

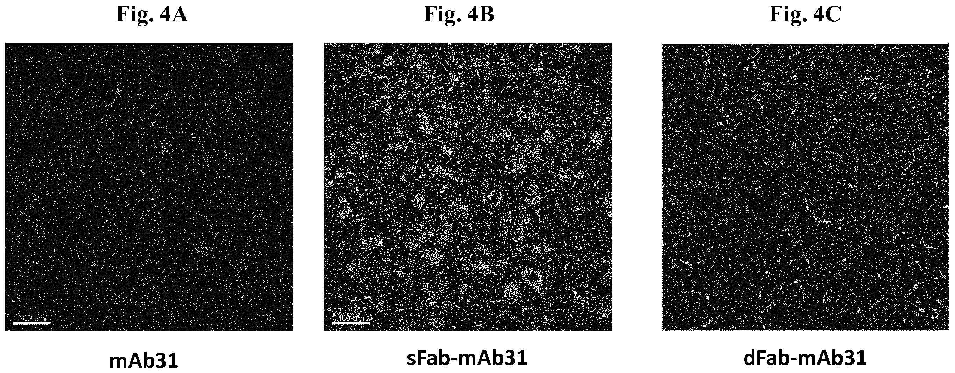

Fig. 4: Plaque decoration of anti-Aβ monoclonal antibody mAb31 (Fig. 4A), single Fab mAb31 (single scFab fused to the C-terminal end of mAb31) (Fig. 4B) and double Fab mAb31 (double scFabs fused to the C-terminal end of mAb31) (Fig. 4C). Construct injected in PS2APP mice (n=3/construct), single intravenous dose 10 mg/kg and then brain perfusion 8 hours post dose. Analysis included immunohistochemistry and confocal microscopy for plaque binding. Data shows that only the single Fab-mAb31 construct are able to cross the BBB and bind to the plaques. The figure shows one representative area of the brain from all animals.

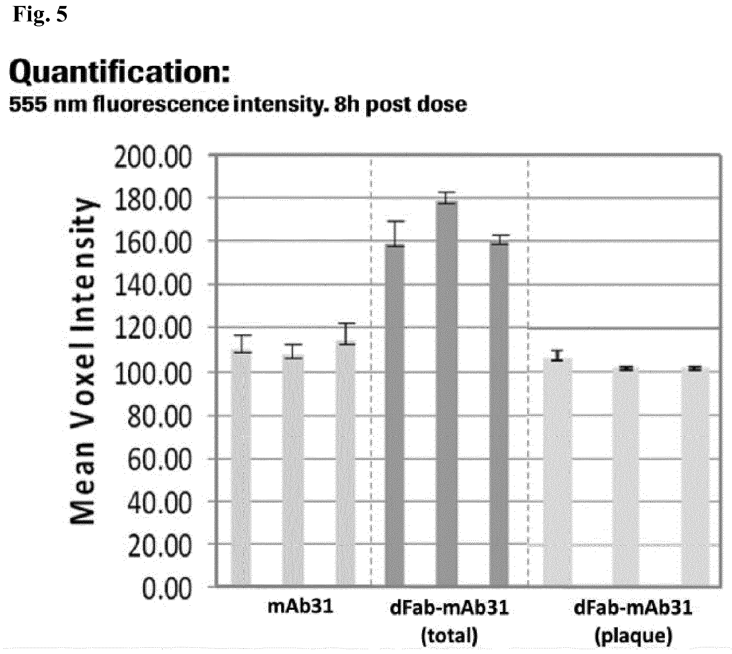

Fig. 5: Shows the quantification of the double Fab-mAb31 construct. The plaque and capillary staining was quantified in all three treated animals in three different regions (9 areas in total for each construct). The data shows that there is only an increase in the capillaries for the double Fab-mAb31 construct compared to mAb31. No increased levels of the double Fab-mAb31 at the plaque (inside the brain) were detected. Quantification of mab31 (HEK control) vs double Fab-mab3, 10 mg/kg, 8h post dose.

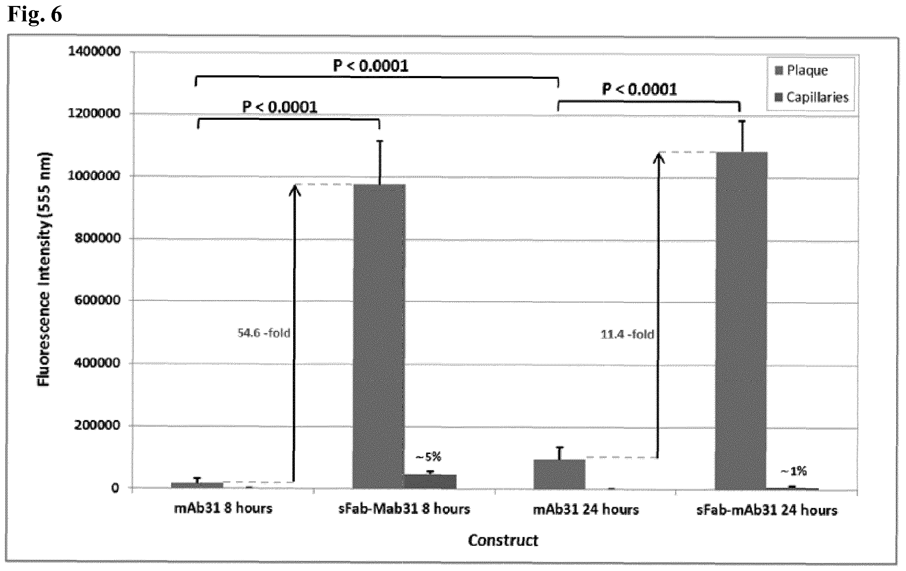

Fig. 6: Shows the quantification of the single Fab-mAb31 construct. The plaque and capillary staining was quantified in all three treated animals in three different regions (9 areas in total for each construct). The data shows that there is a massive increase at the plaques for the single Fab-mAb31 construct compare to mAb31. Quantification of the fluorescence signal indicates more that 50-fold increase of the single Fab-mAb31 compare to the mAb31 construct. There is also a transient staining in the capillaries for the single Fab-mAb31 construct compare to mAb31 indicating the crossing over the BBB. Quantification of mab31 (HEK control) vs single Fab-mab31 10 mg/kg, 8h post dose and 25 mg/kg, 24h post dose.

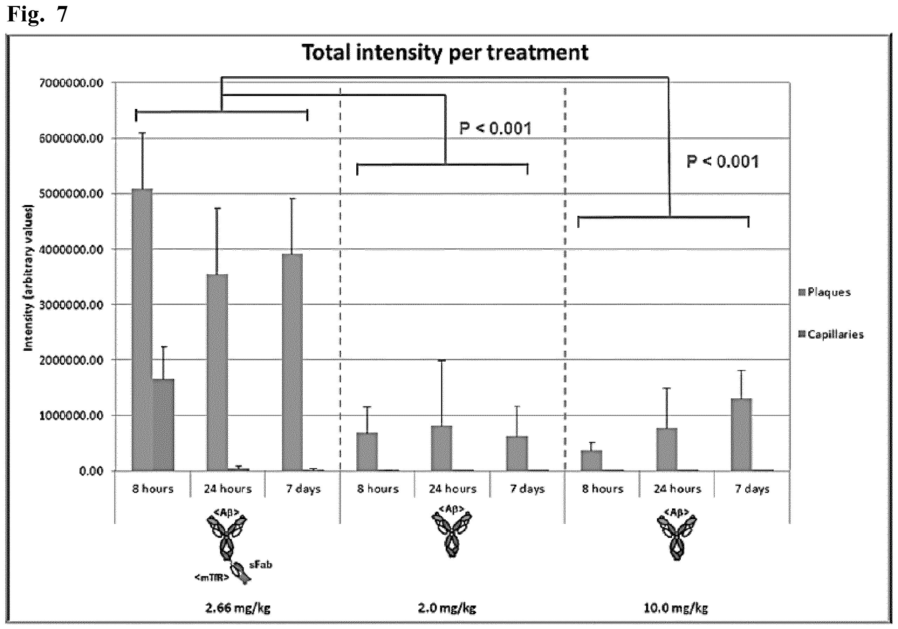

Fig. 7: Plaque decoration of anti-Aβ monoclonal antibody mAb31 at two different doses and single Fab mAb31 (single scFab fused to the C-terminal end of mAb31) at a very low dose. Construct injected in PS2APP mice (n=3/construct), single intravenous dose and then brain perfusion at various time points post dose. Analysis included immunohistochemistry and confocal microscopy for plaque binding. Data shows that only the single Fab-mAb31 construct are able to cross the BBB and bind to the plaques. The brain exposure is very rapid for the single Fab-mAb31 construct and the plaque decoration is sustainable over at least one week from a single administration.

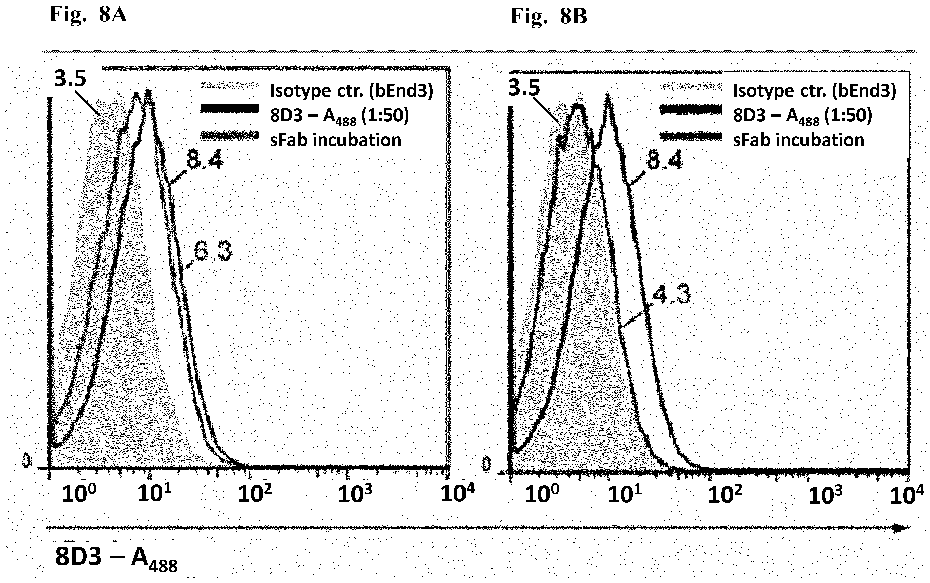

Fig. 8: Quantification of cell surface expression of TfR treated with single Fab-mab31 or double Fab-mAb31. Transferrin receptor (TfR) cell surface down-regulation by the double Fab-mAb31 construct. Brain endothelial cells expressing the TfR were incubated for 24 hours with either the single Fab-mAb31 construct (Fig. 8A) or the double Fab-mAb31 construct (Fig. 8B). Only the double Fab-mAb31 construct lowered the level of cell surface expressed TfR.

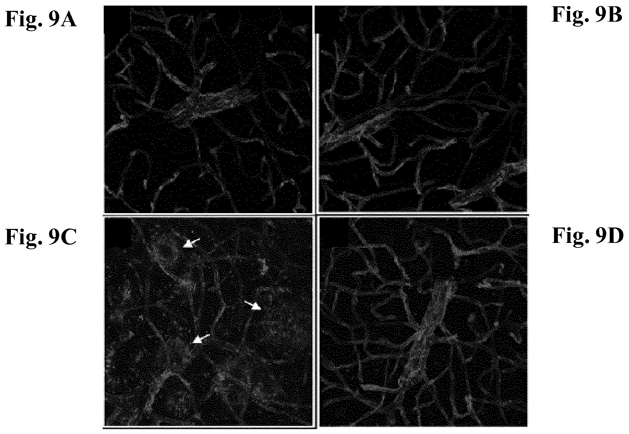

Fig. 9: In vivo cell trafficking of TfR treated with single Fab-mab31 or double Fab-mAb31. Early time points investigating capillary and plaque staining in vivo. Both sFab-MAb31 (Fig. 9A) and dFab-MAb31 (Fig. 9B) decorates the brain vasculature 15 minutes after injection with no difference in their distribution. 8 hours post-injection, sFab-MAb31 reaches the parenchyma and decorates amyloid plaques (arrows, Fig 9C) whereas dFab-MAb31 stays within brain vasculature similarly to the 15 minutes time point (Fig. 9D). No amyloid plaques in the parenchyma are detected for the dFab-MAb31.

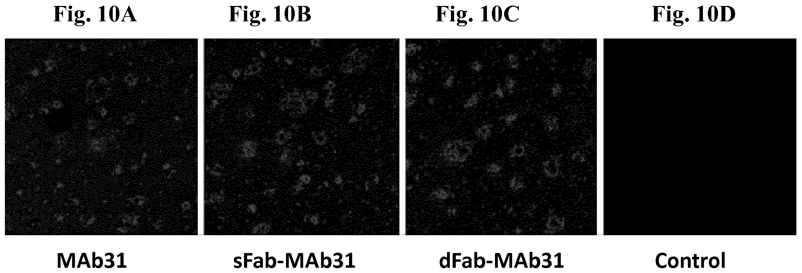

Fig. 10: In vivo cell trafficking of TfR treated with single Fab-mab31 or double Fab-mAb31. To control the integrity of all constructs used in the study, staining of 18 months mouse APP transgenic brain cryosections was done using MAb31 (Fig. 10A), sFab-MAb31 (Fig. 10B) or dFab-MAB31 (Fig. 10C). Fig. 10D shows the results of the control. Results showed that all 3 constructs detected amyloid plaques in the brain of transgenic mice.

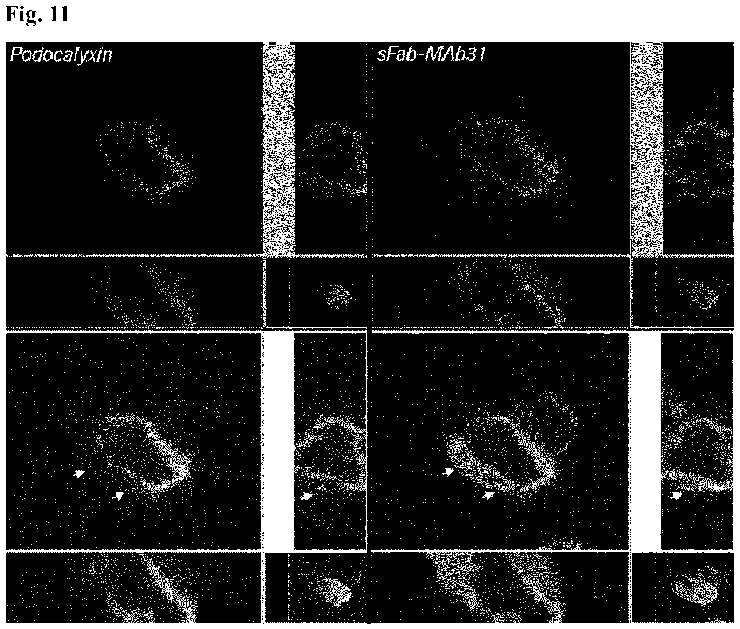

Fig. 11: In vivo cell trafficking of TfR treated with single Fab-mab31. High resolution confocal microscopy on in vivo treated samples shows that sFab-MAb31 do not decorate the luminal side of brain capillaries but are contained within vesicle-like structures crossing the luminal membrane of endothelial cells and within the endothelial cell cytosol. Arrows in Fig 11 indicate vesicles containing sFab-MAb31 constructs on the abluminal side of endothelial cell nuclei. These data suggest that both sFab-MAb31 can enter the brain endothelial cells and cross the vasculature and reach amyloid plaques within the parenchyma space of the brain (Compare with Figure 9A and C).

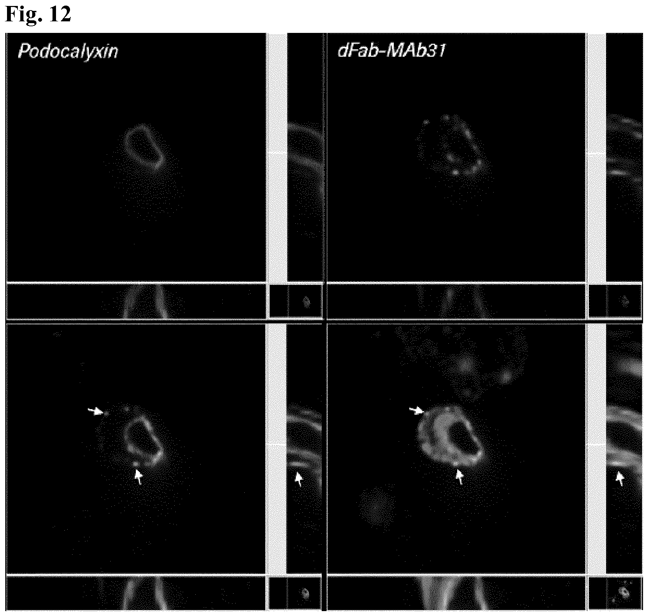

Fig. 12: In vivo cell trafficking of TfR treated with double Fab-mab31. High resolution confocal microscopy on in vivo treated samples shows dFab-MAb31 do not decorate the luminal side of brain capillaries but are contained within vesicle-like structures crossing the luminal membrane of endothelial cells and within the endothelial cell cytosol. Arrows in Fig 12 indicate vesicles containing dFab-MAb31 constructs on the abluminal side of endothelial cell nuclei. These data suggest that dFab-MAb31 can enter the brain endothelial cells but are trapped not able to cross the vasculature and therefore not reach the amyloid plaques within the parenchyma space of the brain (Compare with Figure 9B and D).

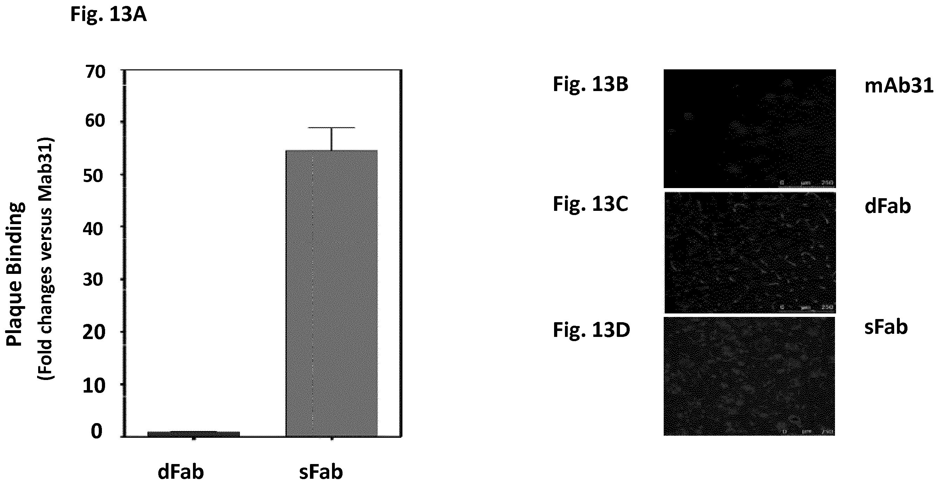

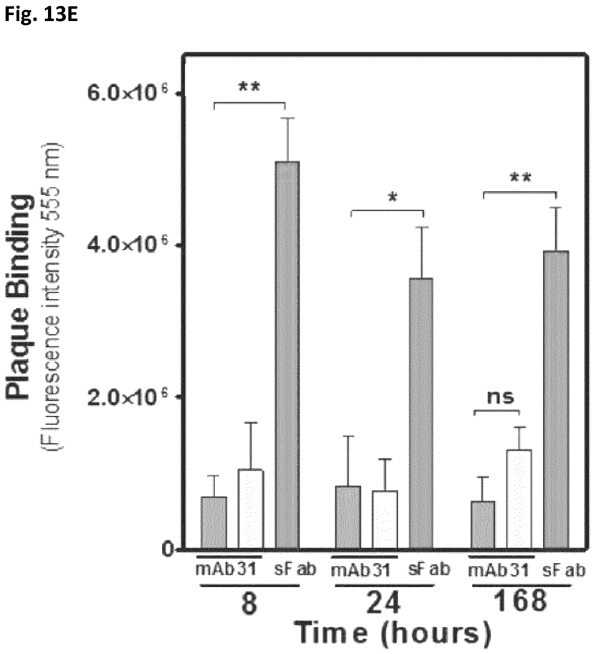

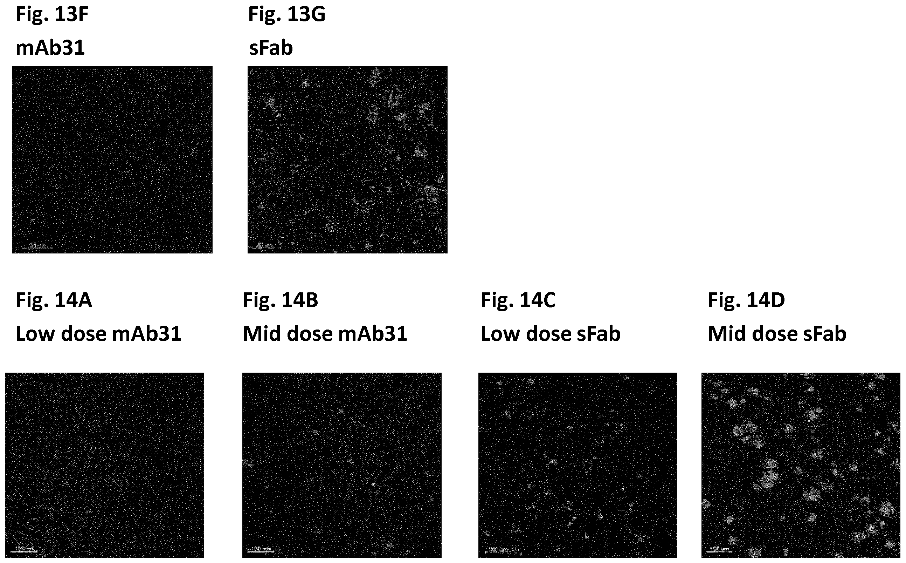

Fig. 13: Brain exposure and plaque decoration after i.v. administration. Fig. 13A: mAb31, dFab and sFab constructs were intravenously injected in PS2APP transgenic animals at 10 mg/kg, animals were perfused and sacrificed 8 hours post injection. No significant increase in plaque decoration was detected for the dFab compared to mAb31. For the sFab construct a 55-fold higher plaque decoration was detected than the parent mAb31 based on fluorescence intensity at 555 nm from the detection antibody. Representative immunohistochemistry staining in cortex of mAb31 (Fig. 13B), dFab (Fig. 13C) and 2. sFab (Fig. 13D) 8 hour post injection. The dFab shows only microvessel staining while the sFab decorates the amyloid-β plaques extensively. Fig. 13E: Demonstration that a low dose of the sFab construct (2.66 mg/kg) rapidly and significantly reaches the plaques in the brain compared to both 2 mg/kg and 10 mg/kg of mAb31. The target engagement of the sFab construct is sustainable over at least one week post injection. Immunohistochemistry staining shows plaque decoration for mAb31 at 2 mg/kg (Fig. 13F) and sFab at 2.66 mg/kg (Fig. 13G) 7 days post injection.

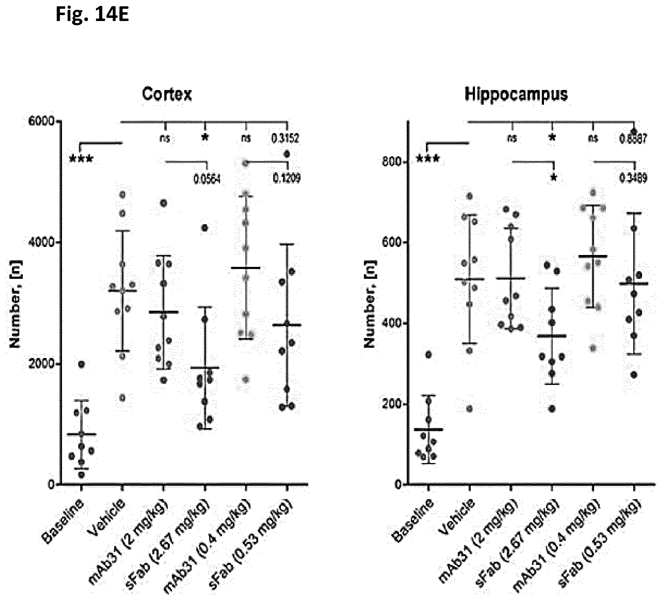

Fig. 14: In vivo efficacy in a chronic study in plaque bearing PS2APP mice treated by 14 weekly i.v. injections. Target plaque binding of administrated constructs bound to residual plaques at the end of the study are shown for low dose mAb31, mid dose mAb31, low dose sFab and mid dose sFab respectively (Fig. 14A-D). Quantitative morphometric analysis after immunohistochemical staining of plaques is shown for cortex and hippocampus (Fig. 14 E). Plaque load of untreated animals sacrificed at an age of 4.5 months is shown as baseline level of amyloidosis at the start of the study. A significant reduction in plaque numbers is evident after treatment with mid dose sFab compared to the progressive plaque formation seen in vehicle treated animals; a trend of reduced plaque formation appears even at the low dose sFab. Thus, sFab construct significantly reduces plaque numbers in both cortex and hippocampus. Analysis of plaque sizes revealed reduction of plaque numbers most pronounced for small plaque sizes. $*p \leq 0.05$, $**p \leq 0.01$, $***p \leq 0.001$.

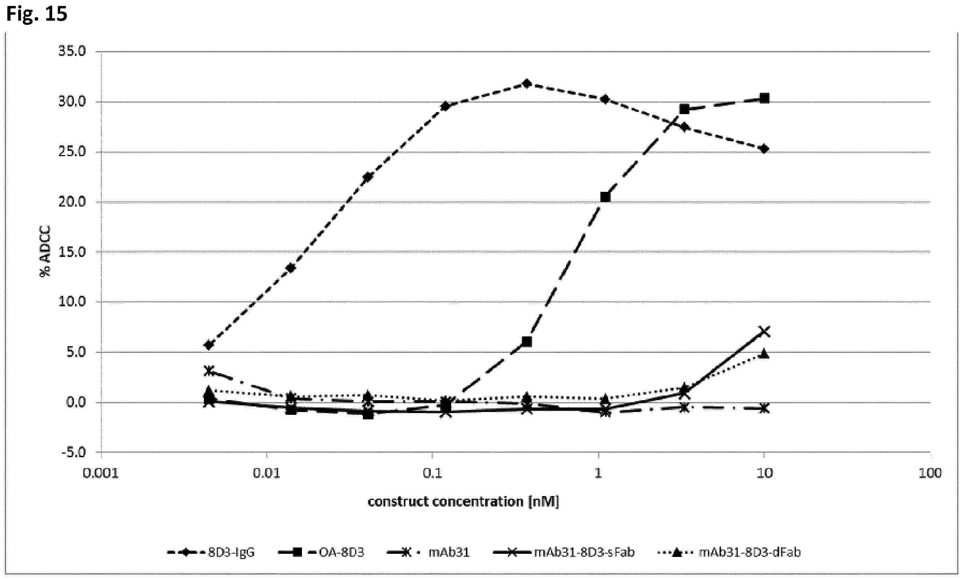

Fig. 15: Antibody multimeric with TfR scFab fragments fused to the Fc C-terminus do not induce ADCC. NK92-mediated killing of BA/F3 mouse erythroleukemia cells was measured by quantifying LDH release. Only multimeric constructs with the TfR-binding Fab moiety in the "conventional" "N-terminal to Fc" orientation induce significant ADCC, while the brain shuttle constructs in reverse orientation are silent.

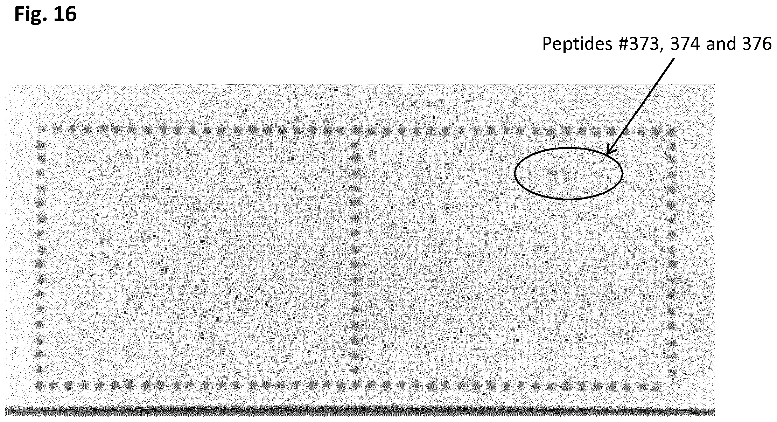

Fig. 16: scFab 8D3 directed to the transferrin receptor binds to three distinct peptides in the extracellular domain of mouse transferrin receptor. Binding of antibody 8D3 to 15mer peptides overlapping by three amino acids was revealed by chemiluminescent detection of antibody incubated on a CelluSpot slide carrying immobilized mTfR peptides. Box: Peptides #373, 374 and 376 bound by 8D3 (Seq. Id. No. 15, 16 and 17).

## DETAILED DESCRIPTION OF EMBODIMENTS OF THE INVENTION

## DEFINITIONS

The "blood-brain barrier" or "BBB" refers to the physiological barrier between the peripheral circulation and the brain and spinal cord which is formed by tight junctions within the brain capillary endothelial plasma membranes, creating a tight barrier that restricts the transport of molecules into the brain, even very small molecules such as urea (60 Daltons). The BBB within the brain, the blood-spinal cord barrier within the spinal cord, and the blood-retinal barrier within the retina are contiguous capillary barriers within the CNS, and are herein collectively referred to as the blood-brain barrier or BBB. The BBB also encompasses the blood-CSF barrier (choroid plexus) where the barrier is comprised of ependymal cells rather than capillary endothelial cells.

The knobs into holes dimerization modules and their use in antibody engineering are described in Carter P.; Ridgway J.B.B.; Presta L.G.: Immunotechnology, Volume 2, Number 1, February 1996, pp. 73-73(1)).

The "central nervous system" or "CNS" refers to the complex of nerve tissues that control bodily function, and includes the brain and spinal cord.

A "blood-brain barrier receptor" (abbreviated "R/BBB" herein) is an extracellular membrane-linked receptor protein expressed on brain endothelial cells which is capable of transporting molecules across the BBB or be used to transport exogenous administrated molecules. Examples of R/BBB herein include: transferrin receptor (TfR), insulin receptor, insulin-like growth factor receptor (IGF-R), low density lipoprotein receptors including without limitation low density lipoprotein receptor-related protein 1 (LRP1) and low density lipoprotein receptor-related protein 8 (LRP8), and heparin-binding epidermal growth factor-like growth factor (HB-EGF). An exemplary R/BBB herein is transferrin receptor (TfR).

The "brain effector entity" refers to a molecule that is to be transported to the brain across the BBB. The effector entity typically has a characteristic therapeutic activity that is desired to be delivered to the brain. Effector entities include neurologically disorder drugs and cytotoxic agents such as e.g. peptides, proteins and antibodies, in particular monoclonal antibodies or fragments thereof directed to a brain target.

The "monovalent binding entity" refers to a molecule able to bind specifically and in a monovalent binding mode to an R/BBB. The blood brain shuttle and/or conjugate of the present invention are characterized by the presence of a single unit of a monovalent binding entity i.e. the blood brain shuttle and/or conjugate of the present invention comprise one unit of the monovalent binding entity. The monovalent binding entity of the disclosure includes but is not limited to proteins, polypeptides, peptides and antibody fragments including Fab, Fab', Fv fragments, single-chain antibody molecules such as e.g. single chain Fab, scFv. The monovalent binding entity can for example be a scaffold protein engineered using state of the art technologies like phage display or immunization. The monovalent binding entity can also be a peptide. The monovalent binding entity of the invention comprises one single chain Fab (scFab). In certain embodiments, the monovalent binding entity comprises a CH2-CH3 Ig domain and a single chain Fab (scFab) directed to a blood brain barrier receptor. The scFab is coupled to the C-terminal end of the CH2-CH3 Ig domain by a linker. In certain embodiments, the scFab is directed to the transferrin receptor.

The "monovalent binding mode" refers to a specific binding to the R/BBB where the interaction between the monovalent binding entity and the R/BBB take place through one single epitope. The monovalent binding mode prevents any dimerization/multimerization of the R/BBB due to a single epitope interaction point. The monovalent binding mode prevents that the intracellular sorting of the R/BBB is changed.

The term "epitope" includes any polypeptide determinant capable of specific binding to an antibody. In certain embodiments, epitope determinant includes chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody.

The "transferrin receptor" ("TfR") is a transmembrane glycoprotein (with a molecular weight of about 180,000) composed of two disulphide-bonded sub-units (each of apparent molecular weight of about 90,000) involved in iron uptake in vertebrates. In one embodiment, the TfR herein is human TfR comprising the amino acid sequence as in Schneider et al. Nature 311 : 675 - 678 (1984), for example.

A "neurological disorder" as used herein refers to a disease or disorder which affects the CNS and/or which has an etiology in the CNS. Exemplary CNS diseases or disorders include, but are not limited to, neuropathy, amyloidosis, cancer, an ocular disease or disorder, viral or microbial infection, inflammation, ischemia, neurodegenerative disease, seizure, behavioral disorders, and a lysosomal storage disease. For the purposes of this application, the CNS will be understood to include the eye, which is normally sequestered from the rest of the body by the blood-retina barrier. Specific examples of neurological disorders include, but are not limited to, neurodegenerative diseases (including, but not limited to, Lewy body disease, postpoliomyelitis syndrome, Shy-Draeger syndrome, olivopontocerebellar atrophy, Parkinson's disease, multiple system atrophy, striatonigral degeneration, tauopathies (including, but not limited to, Alzheimer disease and supranuclear palsy), prion diseases (including, but not limited to, bovine spongiform encephalopathy, scrapie, Creutzfeldt-Jakob syndrome, kuru, Gerstmann-Straussler-Scheinker disease, chronic wasting disease, and fatal familial insomnia), bulbar palsy, motor neuron disease, and nervous system heterodegenerative disorders (including, but not limited to, Canavan disease, Huntington's disease, neuronal ceroid- lipofuscinosis, Alexander's disease, Tourette's syndrome, Menkes kinky hair syndrome, Cockayne syndrome, Halervorden-Spatz syndrome, Iafora disease, Rett syndrome, hepatolenticular degeneration, Lesch-Nyhan syndrome, and Unverricht-Lundborg syndrome), dementia (including, but not limited to, Pick's disease, and spinocerebellar ataxia), cancer (e.g. of the CNS and/or brain, including brain metastases resulting from cancer elsewhere in the body).

A "neurological disorder drug" is a drug or therapeutic agent that treats one or more neurological disorder(s). Neurological disorder drugs of the invention include, but are not limited to, small molecule compounds, antibodies, peptides, proteins, natural ligands of one or more CNS target(s), modified versions of natural ligands of one or more CNS target(s), aptamers, inhibitory nucleic acids (i.e., small inhibitory RNAs (siRNA) and short hairpin RNAs (shRNA)), ribozymes, and small molecules, or active fragments of any of the foregoing. Exemplary neurological disorder drugs of the invention are described herein and include, but are not limited to: antibodies, aptamers, proteins, peptides, inhibitory nucleic acids and small molecules and active fragments of any of the foregoing that either are themselves or specifically recognize and/or act upon (i.e., inhibit, activate, or detect) a CNS antigen or target molecule such as, but not limited to, amyloid precursor protein or portions thereof, amyloid beta, beta-secretase, gamma-secretase, tau, alpha-synuclein, parkin, huntingtin, DR6, presenilin, ApoE, glioma or other CNS cancer markers, and neurotrophins. Non-limiting examples of neurological disorder drugs and the corresponding disorders they may be used to treat: Brain-derived neurotrophic factor (BDNF), Chronic brain injury (Neurogenesis), Fibroblast growth factor 2 (FGF-2), Anti-Epidermal Growth Factor Receptor Brain cancer, (EGFR)-antibody, Glial cell-line derived neural factor Parkinson's disease, (GDNF), Brain-derived neurotrophic factor (BDNF) Amyotrophic lateral sclerosis, depression, Lysosomal enzyme Lysosomal storage disorders of the brain, Ciliary neurotrophic factor (CNTF) Amyotrophic lateral sclerosis, Neuregulin-1 Schizophrenia, Anti-HER2 antibody (e.g. trastuzumab) Brain metastasis from HER2 -positive cancer.

An "imaging agent" is a compound that has one or more properties that permit its presence and/or location to be detected directly or indirectly. Examples of such imaging agents include proteins and small molecule compounds incorporating a labeled entity that permits detection.

A "CNS antigen" or "brain target" is an antigen and/or molecule expressed in the CNS, including the brain, which can be targeted with an antibody or small molecule. Examples of such antigen and/or molecule include, without limitation: beta-secretase 1 (BACEI), amyloid beta (Abeta), epidermal growth factor receptor (EGFR), human epidermal growth factor receptor 2 (HER2), Tau, apolipoprotein E4 (ApoE4), alpha-synuclein, CD20, huntingtin, prion protein (PrP), leucine rich repeat kinase 2 (LRRK2), parkin, presenilin 1, presenilin 2, gamma secretase, death receptor 6 (DR6), amyloid precursor protein (APP), p75 neurotrophin receptor (p75NTR), and caspase 6. In one embodiment, the antigen is BACE1.

A "native sequence" protein herein refers to a protein comprising the amino acid sequence of a protein found in nature, including naturally occurring variants of the protein. The term as used herein includes the protein as isolated from a natural source thereof or as recombinantly produced.

The term "antibody" herein is used in the broadest sense and specifically covers monoclonal antibodies, polyclonal antibodies, multispecific antibodies {e.g. bispecific antibodies) formed from at least two intact antibodies, and antibody fragments so long as they exhibit the desired biological activity.

"Antibody fragments" herein comprise a portion of an intact antibody which retains the ability to bind antigen. Examples of antibody fragments include Fab, Fab', F(ab')$_2$, and Fv fragments; diabodies; linear antibodies; single-chain antibody molecules such as e.g. single chain Fab (scFab), scFv and multispecific antibodies formed from antibody fragments. The "Single chain Fab" format is e.g. described in Hust M. et al. BMC Biotechnol. 2007 Mar 8;7:14.

The term "monoclonal antibody" as used herein refers to an antibody obtained from a population of substantially homogeneous antibodies, i.e., the individual antibodies comprising the population are identical and/or bind the same epitope, except for possible variants that may arise during production of the monoclonal antibody, such variants generally being present in minor amounts. In contrast to polyclonal antibody preparations that typically include different antibodies directed against different determinants (epitopes), each monoclonal antibody is directed against a single determinant on the antigen. In addition to their specificity, the monoclonal antibodies are advantageous in that they are uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example. Specific examples of monoclonal antibodies herein include chimeric antibodies, humanized antibodies, and human antibodies, including antigen-binding fragments thereof. The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al, Proc. Natl. Acad. Sci. USA, 81 :6851-6855 (1984)). Chimeric antibodies of interest herein include "primatized" antibodies comprising variable domain antigen-binding sequences derived from a non-human primate {e.g. Old World Monkey, such as baboon, rhesus or cynomolgus monkey) and human constant region sequences (US Pat No. 5,693,780).

"Humanized" forms of non-human {e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence, except for FR substitution(s) as noted above. The humanized antibody optionally also will comprise at least a portion of an immunoglobulin constant region, typically that of a human immunoglobulin. For further details, see Jones et al, Nature 321 :522-525 (1986); Riechmann et al, Nature 332:323-329 (1988); and Presta, Curr. Op. Struct. Biol 2:593-596 (1992).

A "human antibody" herein is one comprising an amino acid sequence structure that corresponds with the amino acid sequence structure of an antibody obtainable from a human B- cell, and includes antigen-binding fragments of human antibodies. Such antibodies can be identified or made by a variety of techniques, including, but not limited to: production by transgenic animals {e.g., mice) that are capable, upon immunization, of producing human antibodies in the absence of endogenous immunoglobulin production (see, e.g., Jakobovits et al, Proc. Natl Acad. Sci. USA, 90:2551 (1993); Jakobovits et al, Nature, 362:255-258 (1993); Bruggermann et al, Year in Immuno., 7:33 (1993); and US Patent Nos. 5,591,669, 5,589,369 and 5,545,807)); selection from phage display libraries expressing human antibodies or human antibody fragments (see, for example, McCafferty et al, Nature 348:552-553 (1990); Johnson et al, Current Opinion in Structural Biology 3:564-571 (1993); Clackson et al, Nature, 352:624-628 (1991); Marks et al, J. Mol. Biol. 222:581-597 (1991); Griffith et al, EMBO J. 12:725-734 (1993);US Patent Nos. 5,565,332 and 5,573,905); generation via in vitro activated B cells (see US Patents 5,567,610 and 5,229,275); and isolation from human antibody producing hybridomas.

A "multispecific antibody" herein is an antibody having binding specificities for at least two different epitopes. Exemplary multispecific antibodies may bind both an R/BBB and a brain antigen. Multispecific antibodies can be prepared as full-length antibodies or antibody fragments (e.g. F(ab')2 bispecific antibodies). Engineered antibodies with two, three or more (e.g. four) functional antigen binding sites are also contemplated (see, e.g., US Appln No. US 2002/0004587 A1, Miller et al.). Multispecific antibodies can be prepared as full length antibodies or antibody fragments.

Antibodies herein include "amino acid sequence variants" with altered antigen-binding or biological activity. Examples of such amino acid alterations include antibodies with enhanced affinity for antigen (e.g. "affinity matured" antibodies), and antibodies with altered Fc region, if present, e.g. with altered (increased or diminished) antibody dependent cellular cytotoxicity (ADCC) and/or complement dependent cytotoxicity (CDC) (see, for example, WO 00/42072, Presta, L. and WO 99/51642, Iduosogie et al); and/or increased or diminished serum half-life (see, for example, WO00/42072, Presta, L.).

An "affinity modified variant" has one or more substituted hypervariable region or framework residues of a parent antibody (e.g. of a parent chimeric, humanized, or human antibody) that alter (increase or reduce) affinity. In one embodiment, the resulting variant(s) selected for further development will have reduced affinity for the R/BBB according to the present invention. A convenient way for generating such substitutional variants uses phage display. Briefly, several hypervariable region sites (e.g. 6-7 sites) are mutated to generate all possible amino substitutions at each site. The antibody variants thus generated are displayed in a monovalent fashion from filamentous phage particles as fusions to the gene III product of M1 3 packaged within each particle. The phage-displayed variants are then screened for their biological activity (e.g. binding affinity). In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and its target. Such contact residues and neighboring residues are candidates for substitution according to the techniques elaborated herein. Once such variants are generated, the panel of variants is subjected to screening and antibodies with altered affinity may be selected for further development.

The antibody herein may be a "glycosylation variant" such that any carbohydrate attached to the Fc region, if present, is altered. For example, antibodies with a mature carbohydrate structure that lacks fucose attached to an Fc region of the antibody are described in US Pat Appl No US 2003/0157108 (Presta, L.). See also US 2004/0093621 (Kyowa Hakko Kogyo Co., Ltd). Antibodies with a bisecting N-acetylglucosamine (GlcNAc) in the carbohydrate attached to an Fc region of the antibody are referenced in WO 2003/011878, Jean-Mairet et al. and US Patent No. 6,602,684, Umana et al. Antibodies with at least one galactose residue in the oligosaccharide attached to an Fc region of the antibody are reported in WO 1997/30087, Patel et al. See, also, WO 1998/58964 (Raju, S.) and WO 1999/22764 (Raju, S.) concerning antibodies with altered carbohydrate attached to the Fc region thereof. See also US 2005/0123546 (Umana et al.) describing antibodies with modified glycosylation. The term "hypervariable region" when used herein refers to the amino acid residues of an antibody that are responsible for antigen binding. The hypervariable region comprises amino acid residues from a "complementarity determining region" or "CDR" (e.g. residues 24- 34 (LI), 50-56 (L2) and 89-97 (L3) in the light chain variable domain and 31-35 (HI), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (e.g. residues 26-32 (LI), 50-52 (L2) and 91-96 (L3) in the light chain variable domain and 26-32 (HI), 53-55 (H2) and 96-101 (H3) in the heavy chain variable domain; Chothia and Lesk J. Mol. Biol. 196:901- 917 (1987)). "Framework" or "FR" residues are those variable domain residues other than the hypervariable region residues as herein defined.

A "full length antibody" is one which comprises an antigen-binding variable region as well as a light chain constant domain (CL) and heavy chain constant domains, CHI, CH2 and CH3. The constant domains may be native sequence constant domains (e.g. human native sequence constant domains) or amino acid sequence variants thereof.

A "naked antibody" is an antibody (as herein defined) that is not conjugated to a heterologous molecule, such as a cytotoxic entity, polymer, or radiolabel.

Antibody "effector functions" refer to those biological activities attributable to the Fc region (a native sequence Fc region or amino acid sequence variant Fc region) of an antibody. Examples of antibody effector functions include Clq binding, complement dependent cytotoxicity (CDC), Fc receptor binding, antibody-dependent cell-mediated cytotoxicity (ADCC), etc. In one embodiment, the antibody herein essentially lacks effector function.

The term "antibody-dependent cellular cytotoxicity (ADCC)" refers to lysis of human target cells by an antibody in the presence of effector cells. The term "complement-dependent cytotoxicity (CDC)" denotes a process initiated by binding of complement factor C1q to the Fc part of most IgG antibody subclasses. Binding of C1q to an antibody is caused by defined protein-protein interactions at the so called binding site. Such Fc part binding sites are known in the state of the art. Such Fc part binding sites are, e.g., characterized by the amino acids L234, L235, D270, N297, E318, K320, K322, P331, and P329 (numbering according to EU index of Kabat). Antibodies of subclass IgG1, IgG2, and IgG3 usually show complement activation including C1q and C3 binding, whereas IgG4 does not activate the complement system and does not bind C1q and/or C3.

Depending on the amino acid sequence of the constant domain of their heavy chains, full length antibodies can be assigned to different "classes". There are five major classes of full length antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called alpha, delta, epsilon, gamma, and mu, respectively. The subunit structures and three-dimensional configurations of different classes of immunoglobulins are well known. The term "recombinant antibody", as used herein, refers to an antibody (e.g. a chimeric, humanized, or human antibody or antigen-binding fragment thereof) that is expressed by a recombinant host cell comprising nucleic acid encoding the antibody. Examples of "host cells" for producing recombinant antibodies include: (1) mammalian cells, for example, Chinese Hamster Ovary (CHO), COS, myeloma cells (including Y0 and NSO cells), baby hamster kidney (BHK), Hela and Vero cells; (2) insect cells, for example, sf9, sf21 and Tn5; (3) plant cells, for example plants belonging to the genus Nicotiana (e.g. Nicotiana tabacum); (4) yeast cells, for example, those belonging to the genus Saccharomyces (e.g. Saccharomyces cerevisiae) or the genus Aspergillus (e.g. Aspergillus niger); (5) bacterial cells, for example Escherichia, coli cells or Bacillus subtilis cells, etc.

As used herein, "specifically binding" or "binds specifically to" refers to an antibody selectively or preferentially binding to an antigen. The binding affinity is generally determined using a standard assay, such as Scatchard analysis, or surface plasmon resonance technique (e.g. using BIACORE®).

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more.

The term "cytotoxic agent" as used herein refers to a substance that inhibits or prevents a cellular function and/or causes cell death or destruction. Cytotoxic agents include, but are not limited to, radioactive isotopes (e.g., At211, 1131, 1125, Y90, Re186, Re188, Sm153, Bi212, P32, Pb212 and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof.

An "effective amount" of an agent, e.g., a pharmaceutical formulation, refers to an amount effective, at dosages and for periods of time necessary, to achieve the desired therapeutic or prophylactic result.

The term "Fc region" herein is used to define a C-terminal region of an immunoglobulin heavy chain that contains at least a portion of the constant region. The Fc region comprises the CH2 and CH3 domains of an immunoglobulin. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The term "CH2-CH3 Ig entity" as used herein refers to a protein entity derived from immunoglobulin CH2 or CH3 domains. The "CH2-CH3 Ig entity" comprises two "CH2-CH3" polypeptides forming a dimer. The immunoglobulin can be IgG, IgA, IgD, IgE or IgM. In one embodiment, the CH2-CH3 Ig entity derived from an IgG immunoglobulin and is referred to herein as "CH2-CH3 IgG entity". The term includes native sequence of CH2-CH3 domains and variant CH2-CH3 domains. In one embodiment, the "CH2-CH3 Ig entity" derives from human heavy chain CH2-CH3 IgG domain which extends from Cys226, or from Pro230, to the carboxyl-terminus of the heavy chain. However, the C-terminal lysine (Lys447) of the Fc region may or may not be present. Unless otherwise specified herein, numbering of amino acid residues in the CH2-CH3 domain region or constant region is according to the EU numbering system, also called the EU index, as described in Kabat et al., Sequences of Proteins of Immunological Interest, 5th Ed. Public Health Service, National Institutes of Health, Bethesda, MD, 1991.

An "conjugate" is fusion protein of the present invention conjugated to one or more heterologous molecule(s), including but not limited to a label, neurological disorder drug or cytotoxic agent.

A " linker" as used herein refers to a chemical linker or a single chain peptide linker that covalently connects the different entities of the blood brain barrier shuttle and/or the fusion protein and/or the conjugate of the present invention. The linker connects for example the brain effector entity to the monovalent binding entity. For example, if the monovalent binding entity comprises a CH2-CH3 Ig entity and a scFab directed to the blood brain barrier receptor, then the linker connects the scFab to the C-terminal end of the CH3-CH2 Ig entity. The linker connecting the brain effector entity to the monovalent binding entity (first linker) and the linker connecting the scFab to the C-terminal end of the CH2-CH3 Ig domain (second linker) can be the same or different.

Single chain peptide linkers, comprised of from one to twenty amino acids joined by peptide bonds, can be used. In certain embodiments, the amino acids are selected from the twenty naturally-occurring amino acids. In certain other embodiments, one or more of the amino acids are selected from glycine, alanine, proline, asparagine, glutamine and lysine. In other embodiments, the linker is a chemical linker. In certain embodiments, said linker is a single chain peptide with an amino acid sequence with a length of at least 25 amino acids, preferably with a length of 32 to 50 amino acids. In one embodiment said linker is (GxS)n with G = glycine, S = serine, (x =3, n= 8, 9 or 10 and m= 0, 1, 2 or 3) or (x = 4 and n= 6, 7 or 8 and m= 0, 1, 2 or 3), preferably with x = 4, n= 6 or 7 and m= 0, 1, 2 or 3, more preferably with x = 4, n= 7 and m= 2. In one embodiment said linker is $(G_4S)_4$ (Seq. Id. No. 17). In one embodiment said linker is $(G_4S)_6G_2$

(Seq. Id. No. 13).

Conjugation may be performed using a variety of chemical linkers. For example, the monovalent binding entity or the fusion protein and the brain effector entity may be conjugated using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p- azidobenzoyl) hexanediamine), bis-diazonium derivatives (such as bis-(p-diazoniumbenzoyl)- ethylenediamine), diisocyanates (such as toluene 2,6-diisocyanate), and bis-active fluorine compounds (such as 1,5-difluoro-2,4-dinitrobenzene). The linker may be a "cleavable linker" facilitating release of the effector entity upon delivery to the brain. For example, an acid- labile linker, peptidase-sensitive linker, photolabile linker, dimethyl linker or disulfide- containing linker (Chari et al, Cancer Res. 52: 127-131 (1992); U.S. Patent No. 5,208,020) may be used.

Covalent conjugation can either be direct or via a linker. In certain embodiments, direct conjugation is by construction of a protein fusion (i.e., by genetic fusion of the two genes encoding the monovalent binding entity towards the R/BBB and effector entity and expressed as a single protein). In certain embodiments, direct conjugation is by formation of a covalent bond between a reactive group on one of the two portions of the monovalent binding entity against the R/BBB and a corresponding group or acceptor on the brain effector entity. In certain embodiments, direct conjugation is by modification (i.e., genetic modification) of one of the two molecules to be conjugated to include a reactive group (as non-limiting examples, a sulfhydryl group or a carboxyl group) that forms a covalent attachment to the other molecule to be conjugated under appropriate conditions. As one non-limiting example, a molecule (i.e., an amino acid) with a desired reactive group (i.e., a cysteine residue) may be introduced into, e.g., the monovalent binding entity towards the R/BBB antibody and a disulfide bond formed with the neurological drug. Methods for covalent conjugation of nucleic acids to proteins are also known in the art (i.e., photocrosslinking, see, e.g., Zatsepin et al. Russ. Chem. Rev. 74: 77-95 (2005)) Conjugation may also be performed using a variety of linkers. For example, a monovalent binding entity and a effector entity may be conjugated using a variety of bifunctional protein coupling agents such as N- succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p- azidobenzoyl) hexanediamine), bis-diazonium derivatives (such as bis-(p-diazoniumbenzoyl)- ethylenediamine), diisocyanates (such as toluene 2,6-diisocyanate), and bis-active fluorine compounds (such as 1,5-difluoro-2,4-dinitrobenzene). Peptide linkers, comprised of from one to twenty amino acids joined by peptide bonds, may also be used. In certain such embodiments, the amino acids are selected from the twenty naturally-occurring amino acids. In certain other such embodiments, one or more of the amino acids are selected from glycine, alanine, proline, asparagine, glutamine and lysine. The linker may be a "cleavable linker" facilitating release of the effector entity upon delivery to the brain. For example, an acid- labile linker, peptidase-sensitive linker, photolabile linker, dimethyl linker or disulfide- containing linker (Chari et al, Cancer Res. 52: 127-131 (1992); U.S. Patent No. 5,208,020) may be used.

A "label" is a marker coupled with the fusion protein herein and used for detection or imaging. Examples of such labels include: radiolabel, a fluorophore, a chromophore, or an affinity tag. In one embodiment, the label is a radiolabel used for medical imaging, for example tc99m or 1123, or a spin label for nuclear magnetic resonance (NMR) imaging (also known as magnetic resonance imaging, mri), such as iodine-123 again, iodine-131, indium- 111, fluorine-19, carbon- 13, nitrogen-15, oxygen- 17, gadolinium, manganese, iron, etc. An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al, J. Chromatogr. B 848:79-87 (2007).

The term "package insert" is used to refer to instructions customarily included in commercial packages of therapeutic products, that contain information about the indications, usage, dosage, administration, combination therapy, contraindications and/or warnings concerning the use of such therapeutic products.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

COMPOSITIONS AND METHODS

The methods and articles of manufacture of the present disclosure use, or incorporate, a blood brain barrier shuttle and/or fusion protein that binds to an R/BBB. The R/BBB antigen to be used for production of, or screening for, monovalent binding entity may be, e.g., a soluble form of or a portion thereof (e.g. the extracellular domain), containing the desired epitope. Alternatively, or additionally, cells expressing BBB- R at their cell surface can be used to generate, or screen for, monovalent binding entity. Other forms of R/BBB useful for generating monovalent binding entity will be apparent to those skilled in the art. Examples of R/BBB herein include transferrin receptor (TfR), insulin receptor, insulin-like growth factor receptor (IGF-R), low density lipoprotein receptor-related protein 1 (LRP1) and LRP8 and heparin-binding epidermal growth factor-like growth factor (HB-EGF).

According to the present invention, a "monovalent binding" entity against TfR is selected based on the data herein demonstrating that such monovalent binding entity display improved CNS (for example, brain) uptake. In order to identify such binding entity, various assays for measuring monovalent binding mode are available including, without limitation: Scatchard assay and surface plasmon resonance technique (e.g. using BIACORE®) and in vivo investigations described herein.

The disclosure provides a method of making a monovalent binding entity useful for transporting a brain effector entity such as e.g. a neurological disorder drug, across the blood-brain barrier comprising selecting a monovalent binding entity from a panel of monovalent binding moieties against an R/BBB because it has an monovalent binding mode for the R/BBB. The monovalent binding mode ensures efficient BBB crossing for certain R/BBB by not interfering with the receptors normal intracellular sorting.

For a neuropathy disorder, a neurological drug may be selected that is an analgesic including, but not limited to, a narcotic/opioid analgesic (i.e., morphine, fentanyl, hydrocodone, meperidine, methadone, oxymorphone, pentazocine, propoxyphene, tramadol, codeine and oxycodone), a nonsteroidal anti-inflammatory drug (NSAID) (i.e., ibuprofen, naproxen, diclofenac, diflunisal, etodolac, fenoprofen, flurbiprofen, indomethacin, ketorolac, mefenamic acid, meloxicam, nabumetone, oxaprozin, piroxicam, sulindac, and tolmetin), a corticosteroid (i.e., cortisone, prednisone, prednisolone, dexamethasone, methylprednisolone and triamcinolone), an antimigraine agent (i.e., sumatriptin, almotriptan, frovatriptan, sumatriptan, rizatriptan, eletriptan, zolmitriptan, dihydroergotamine, eletriptan and ergotamine), acetaminophen, a salicylate (i.e., aspirin, choline salicylate, magnesium salicylate, diflunisal, and salsalate), an anti-convulsant (i.e., carbamazepine, clonazepam, gabapentin, lamotrigine, pregabalin, tiagabine, and topiramate), an anaesthetic (i.e., isoflurane, trichloroethylene, halothane, sevoflurane, benzocaine, chloroprocaine, cocaine, cyclomethycaine, dimethocaine, propoxycaine, procaine, novocaine, proparacaine, tetracaine, articaine, bupivacaine, carticaine, cinchocaine, etidocaine, levobupivacaine, lidocaine, mepivacaine, piperocaine, prilocaine, ropivacaine, trimecaine, saxitoxin and tetrodotoxin), and a cox-2-inhibitor (i.e., celecoxib, rofecoxib, and valdecoxib). For a neuropathy disorder with vertigo involvement, a neurological drug may be selected that is an anti-vertigo agent including, but not limited to, meclizine, diphenhydramine, promethazine and diazepam. For a neuropathy disorder with nausea involvement, a neurological drug may be selected that is an anti-nausea agent including, but not limited to, promethazine, chlorpromazine, prochlorperazine, trimethobenzamide, and metoclopramide. For a neurodegenerative disease, a neurological drug may be selected that is a growth hormone or neurotrophic factor; examples include but are not limited to brain-derived neurotrophic factor (BDNF), nerve growth factor (NGF), neurotrophin-4/5, fibroblast growth factor (FGF)-2 and other FGFs, neurotrophin (NT)-3, erythropoietin (EPO), hepatocyte growth factor (HGF), epidermal growth factor (EGF), transforming growth factor (TGF)-alpha, TGF- beta, vascular endothelial growth factor (VEGF), interleukin-1 receptor antagonist (IL-lra), ciliary neurotrophic factor (CNTF), glial-derived neurotrophic factor (GDNF), neurturin, platelet-derived growth factor (PDGF), heregulin, neuregulin, artemin, persephin, interleukins, glial cell line derived neurotrophic factor (GFR), granulocyte-colony stimulating factor (CSF), granulocyte-macrophage-CSF, netrins, cardiotrophin-1, hedgehogs, leukemia inhibitory factor (LIF), midkine, pleiotrophin, bone morphogenetic proteins (BMPs), netrins, saposins, semaphorins, and stem cell factor (SCF). For cancer, a neurological drug may be selected that is a chemotherapeutic agent. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamme, mechlorethamme oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gammaII and calicheamicin omegaII (see, e.g., Agnew, Chem Intl. Ed. Engl, 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino- doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin,methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"- trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., TAXOL® paclitaxel (Bristol-Myers Squibb Oncology, Princeton, N.J.), ABRAXANETM Cremophor-free, albumin-engineered nanoparticle formulation of paclitaxel (American Pharmaceutical Partners, Schaumberg, Illinois), and TAXOTERE® doxetaxel (Rhone-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP- 16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluorometlhylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATINTM) combined with 5-FU and leucovovin.

Also included in this definition of chemotherapeutic agents are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX® tamoxifen), EVISTA® raloxifene, droloxifene, 4-hydroxytamoxifen, trioxifene, keoxifene, LY 117018, onapristone, and FARESTON® toremifene; anti-progesterones; estrogen receptor down-regulators (ERDs); agents that function to suppress or shut down the ovaries, for example, leutinizing hormone-releasing hormone (LHRH) agonists such as LUPRON® and ELIGARD® leuprolide acetate, goserelin acetate, buserelin acetate and tripterelin; other anti- androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole. In addition, such definition of chemotherapeutic agents includes bisphosphonates such as clodronate (for example, BONEFOS® or OSTAC®), DIDROCAL® etidronate, NE-58095, ZOMETA® zoledronic acid/zoledronate, FOSAMAX® alendronate, AREDIA® pamidronate, SKELID® tiludronate, or ACTONEL® risedronate; as well as troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; LURTOTECAN® topoisomerase 1 inhibitor; ABARELLX® rmRH; lapatinib ditosylate (an ErbB-2 and EGFR dual tyrosine kinase small-molecule inhibitor also known as GW572016); and pharmaceutically acceptable salts, acids or derivatives of any of the above.

Another group of compounds that may be selected as neurological drugs for cancer treatment or prevention are anti-cancer immunoglobulins (including, but not limited to, trastuzumab, bevacizumab, alemtuxumab, cetuximab, gemtuzumab ozogamicin, ibritumomab tiuxetan, panitumumab and rituximab). In some instances, antibodies in conjunction with a toxic label may be used to target and kill desired cells (i.e., cancer cells), including, but not limited to, tositumomab with a radiolabel.

For an ocular disease or disorder, a neurological drug may be selected that is an anti- an-giogenic ophthalmic agent (i.e., bevacizumab, ranibizumab and pegaptanib), an ophthalmic glaucoma agent (i.e., carbachol, epinephrine, demecarium bromide, apraclonidine, brimonidine, brinzolamide, levobunolol, timolol, betaxolol, dorzolamide, bimatoprost, carteolol, metipranolol, dipivefrin, travoprost and latanoprost), a carbonic anhydrase inhibitor (i.e., methazolamide and acetazolamide), an ophthalmic antihistamine (i.e., naphazoline, phenylephrine and tetrahydrozoline), an ocular lubricant, an ophthalmic steroid (i.e., fluorometholone, prednisolone, loteprednol, dexamethasone, difluprednate, rimexolone, fluocinolone, medrysone and triamcinolone), an ophthalmic anesthetic (i.e., lidocaine, proparacaine and tetracaine), an ophthalmic anti-infective (i.e., levofloxacin, gatifloxacin, ciprofloxacin, moxif oxacin, chloramphenicol, bacitracin/polymyxin b, sulfacetamide, tobramycin, azithromycin, besifloxacin, norfloxacin, sulfisoxazole, gentamicin, idoxuridine, erythromycin, natamycin, gramicidin, neomycin, ofloxacin, trif uridine, ganciclovir, vidarabine), an ophthalmic anti-inflammatory agent (i.e., nepafenac, ketorolac, flurbiprofen, suprofen, cyclosporine, triamcinolone, diclofenac and bromfenac), and an ophthalmic antihistamine or decongestant (i.e., ketotifen, olopatadine, epinastine, naphazoline, cromolyn, tetrahydrozoline, pemirolast, bepotastine, naphazoline, phenylephrine, nedocromil, lodox amide, phenylephrine, emedastine and azelastine).For a seizure disorder, a neurological drug may be selected that is an anticonvulsant or antiepileptic including, but not limited to, barbiturate anticonvulsants (i.e., primidone, metharbital, mephobarbital, allobarbital, amobarbital, aprobarbital, alphenal, barbital, brallobarbital and phenobarbital), benzodiazepine anticonvulsants (i.e., diazepam, clonazepam, and lorazepam), carbamate anticonvulsants (i.e. felbamate), carbonic anhydrase inhibitor anticonvulsants (i.e., acetazolamide, topiramate and zonisamide), dibenzazepine anticonvulsants (i.e., rufinamide, carbamazepine, and oxcarbazepine), fatty acid derivative anticonvulsants (i.e., divalproex and valproic acid), gamma-aminobutyric acid analogs (i.e., pregabalin, gabapentin and vigabatrin), gamma-aminobutyric acid reuptake inhibitors (i.e., tiagabine), gamma-aminobutyric acid transaminase inhibitors (i.e., vigabatrin), hydantoin anticonvulsants (i.e. phenytoin, ethotoin, fosphenytoin and mephenytoin), miscellaneous anticonvulsants (i.e., lacosamide and magnesium sulfate), progestins (i.e., progesterone), oxazolidinedione anticonvulsants (i.e., paramethadione and trimethadione), pyrrolidine anticonvulsants (i.e., levetiracetam), succinimide anticonvulsants (i.e., ethosuximide and methsuximide), triazine anticonvulsants (i.e., lamotrigine), and urea anticonvulsants (i.e., phenacemide and pheneturide).

For a lysosomal storage disease, a neurological drug may be selected that is itself or otherwise mimics the activity of the enzyme that is impaired in the disease. Exemplary recombinant enzymes for the treatment of lysosomal storage disorders include, but are not limited to those set forth in e.g., U.S. Patent Application publication no. 2005/0142141 (i.e., alpha-L-iduronidase, iduronate-2-sulphatase, N-sulfatase, alpha-N-acetylglucosaminidase, N-acetyl-galactosamine-6-sulfatase, beta-galactosidase, arylsulphatase B, beta-glucuronidase, acid alpha-glucosidase, glucocerebrosidase, alpha-galactosidase A, hexosaminidase A, acid sphingomyelinase, betagalactocerebrosidase, beta-galactosidase, arylsulfatase A, acid ceramidase, aspartoacylase, palmitoyl-protein thioesterase 1 and trip eptidyl amino peptidase 1).

For amyloidosis, a neurological drug may be selected that includes, but is not limited to, an antibody or other binding molecule (including, but not limited to a small molecule, a peptide, an aptamer, or other protein binder) that specifically binds to a target selected from: beta secretase, tau, presenilin, amyloid precursor protein or portions thereof, amyloid beta peptide or oligomers or fibrils thereof, death receptor 6 (DR6), receptor for advanced glycation endproducts (RAGE), parkin, and huntingtin; a cholinesterase inhibitor (i.e., galantamine, donepezil, rivastigmine and tacrine); an NMDA receptor antagonist (i.e., memantine), a monoamine depletor (i.e., tetrabenazine); an ergoloid mesylate; an anticholinergic antiparkinsonism agent (i.e., procyclidine, diphenhydramine, trihexylphenidyl, benztropine, biperiden and trihexyphenidyl); a dopaminergic antiparkinsonism agent (i.e., entacapone, selegiline, pramipexole, bromocriptine, rotigotine, selegiline, ropinirole, rasagiline, apomorphine, carbidopa, levodopa, pergolide, tolcapone and amantadine); a tetrabenazine; an anti-inflammatory (including, but not limited to, a nonsteroidal anti-inflammatory drug (i.e., indomethicin and other compounds listed above); a hormone (i.e., estrogen, progesterone and leuprolide); a vitamin (i.e., folate and nicotinamide); a dimebolin; a homotaurine (i.e., 3- aminopropanesulfonic acid; 3 APS); a serotonin receptor activity modulator (i.e., xaliproden); an, an interferon, and a glucocorticoid.

For a viral or microbial disease, a neurological drug may be selected that includes, but is not limited to, an antiviral compound (including, but not limited to, an adamantane antiviral (i.e., rimantadine and amantadine), an antiviral interferon (i.e., peginterferon alfa-2b), a chemokine receptor antagonist (i.e., maraviroc), an integrase strand transfer inhibitor (i.e., raltegravir), a neuraminidase inhibitor (i.e., oseltamivir and zanamivir), a non-nucleoside reverse transcriptase inhibitor (i.e., efavirenz, etravirine, delavirdine and nevirapine), a nucleoside reverse transcriptase inhibitors (tenofovir, abacavir, lamivudine, zidovudine, stavudine, entecavir, emtricitabine, adefovir, zalcitabine, telbivudine and didanosine), a protease inhibitor (i.e., darunavir, atazanavir, fosamprenavir, tipranavir, ritonavir, nelfmavir, amprenavir, indinavir and saquinavir), a purine nucleoside (i.e., valacyclovir, famciclovir, acyclovir, ribavirin, ganciclovir, valganciclovir and cidofovir), and a miscellaneous antiviral (i.e., enfuvirtide, foscarnet, palivizumab and fomivirsen)), an antibiotic (including, but not limited to, an aminopenicillin (i.e., amoxicillin, ampicillin, oxacillin, nafcillin, cloxacillin, dicloxacillin, flucoxacillin, temocillin, azlocillin, carbenicillin, ticarcillin, mezlocillin, piperacillin and bacampicillin), a cephalosporin (i.e., cefazolin, cephalexin, cephalothin, cefamandole, ceftriaxone, cefotaxime, cefpodoxime, ceftazidime, cefadroxil, cephradine, loracarbef, cefotetan, cefuroxime, cefprozil, cefaclor, and cefoxitin), a carbapenem/penem (i.e., imipenem, meropenem, ertapenem, faropenem and doripenem), a monobactam (i.e., aztreonam, tigemonam, norcardicin A and tabtoxinine-beta-lactam, a beta-lactamase inhibitor (i.e., clavulanic acid, tazobactam and sulbactam) in conjunction with another beta-lactam antibiotic, an aminoglycoside (i.e., amikacin, gentamicin, kanamycin, neomycin, netilmicin, streptomycin, tobramycin, and paromomycin), an ansamycin (i.e., geldanamycin and herbimycin), a carbacephem (i.e., loracarbef), a glycopeptides (i.e., teicoplanin and vancomycin), a macrolide (i.e., azithromycin, clarithromycin, dirithromycin, erythromycin, roxithromycin, troleandomycin, telithromycin and spectinomycin), a monobactam (i.e., aztreonam), a quinolone (i.e., ciprofloxacin, enoxacin, gatifloxacin, levofloxacin, lomefloxacin, moxifloxacin, norfloxacin, ofloxacin, trovafloxacin, grepafloxacin, sparfloxacin and temafloxacin), a sulfonamide (i.e., mafenide, sulfonamidochrysoidine, sulfacetamide, sulfadiazine, sulfamethizole, sulfanilamide, sulfasalazine, sulfisoxazole, trimethoprim, trimethoprim and sulfamethoxazole), a tetracycline (i.e., tetracycline, demeclocycline, doxycycline, minocycline and oxytetracycline), an antineoplastic or cytotoxic antibiotic (i.e., doxorubicin, mitoxantrone, bleomycin, daunorubicin, dactinomycin, epirubicin, idarubicin, plicamycin, mitomycin, pentostatin and valrubicin) and a miscellaneous antibacterial compound (i.e., bacitracin, colistin and polymyxin B)), an antifungal (i.e., metronidazole, nitazoxanide, imidazole, chloroquine, iodoquinol and paromomycin), and an antiparasitic (including, but not limited to, quinine, chloroquine, amodiaquine, pyrimethamine, sulphadoxine, proguanil, mefloquine, atovaquone, primaquine, artemesinin, halofantrine, doxycycline, clindamycin, mebendazole, pyrantel pamoate, thiabendazole, diethylcarbamazine, ivermectin, rifampin, amphotericin B, melarsoprol, efornithine and albendazole). For ischemia, a neurological drug may be selected that includes, but is not limited to, a thrombolytic (i.e., urokinase, alteplase, reteplase and tenecteplase), a platelet aggregation inhibitor (i.e., aspirin, cilostazol, clopidogrel, prasugrel and dipyridamole), a statin (i.e., lovastatin, pravastatin, fiuvastatin, rosuvastatin, atorvastatin, simvastatin, cerivastatin and pitavastatin), and a compound to improve blood flow or vascular flexibility, including, e.g., blood pressure medications.

For a behavioral disorder, a neurological drug may be selected from a behavior- modifying compound including, but not limited to, an atypical antipsychotic (i.e., risperidone, olanzapine, apripiprazole, quetiapine, paliperidone, asenapine, clozapine, iloperidone and ziprasidone), a phenothiazine antipsychotic (i.e., prochlorperazine, chlorpromazine, fluphenazine, perphenazine, trifluoperazine, thioridazine and mesoridazine), a thioxanthene (i.e., thiothixene), a miscellaneous antipsychotic (i.e., pimozide, lithium, molindone, haloperidol and loxapine), a selective serotonin reuptake inhibitor (i.e., citalopram, escitalopram, paroxetine, fluoxetine and sertraline), a serotonin-norepinephrine reuptake inhibitor (i.e., duloxetine, venlafaxine, desvenlafaxine, a tricyclic antidepressant (i.e., doxepin, clomipramine, amoxapine, nortriptyline, amitriptyline, trimipramine, imipramine, protriptyline and desipramine), a tetracyclic antidepressant (i.e., mirtazapine and maprotiline), a phenylpiperazine antidepressant (i.e., trazodone and nefazodone), a monoamine oxidase inhibitor (i.e., isocarboxazid, phenelzine, selegiline and tranylcypromine), a benzodiazepine (i.e., alprazolam, estazolam, flurazeptam, clonazepam, lorazepam and diazepam), a norepinephrine-dopamine reuptake inhibitor (i.e., bupropion), a CNS stimulant (i.e., phentermine, diethylpropion, methamphetamine, dextroamphetamine, amphetamine, methylphenidate, dexmethylphenidate, lisdexamfetamine, modafmil, pemoline, phendimetrazme, benzphetamine, phendimetrazme, armodafmil, diethylpropion, caffeine, atomoxetine, doxapram, and mazindol), an anxiolytic/sedative/hypnotic (including, but not limited to, a barbiturate (i.e., secobarbital, phenobarbital and mephobarbital), a benzodiazepine (as described above), and a miscellaneous anxiolytic/sedative/hypnotic (i.e. diphenhydramine, sodium oxybate, zaleplon, hydroxyzine, chloral hydrate, aolpidem, buspirone, doxepin, eszopiclone, ramelteon, meprobamate and ethclorvynol)), a secretin (see, e.g., Ratliff-Schaub et al. Autism 9: 256-265 (2005)), an opioid peptide (see, e.g., Cowen et al, J. Neurochem. 89:273-285 (2004)), and a neuropeptide (see, e.g., Hethwa et al. Am. J. Physiol. 289: E301-305 (2005)).

For CNS inflammation, a neurological drug may be selected that addresses the inflammation itself (i.e., a non-steroidal anti-inflammatory agent such as ibuprofen or naproxen), or one which treats the underlying cause of the inflammation (i.e., an anti-viral or anti-cancer agent).

In another embodiment, the brain effector entity is an intact or full-length antibody. Depending on the amino acid sequence of the constant domain of their heavy chains, intact antibodies can be assigned to different classes. There are five major classes of intact antibodies: IgA, IgD, IgE, IgG, and IgM, and several of these may be further divided into subclasses (isotypes), e.g., IgG1, IgG2, IgG3, IgG4, IgA, and IgA2. The heavy chain constant domains that correspond to the different classes of antibodies are called $\alpha$, $\delta$, $\epsilon$, $\gamma$, and $\mu$, respectively. The subunit structures and three-dimensional configurations of different classes of immunoglobulins are well known. In one embodiment, the intact antibody lacks effector function.

Techniques for generating antibodies are known and examples provided above in the definitions section of this document. In one embodiment, the antibody is a chimeric, humanized, or human antibody or antigen-binding fragment thereof.

Various techniques are available for determining binding of the monovalent binding entity to the R/BBB. One such assay is an enzyme linked immunosorbent assay (ELIS A) for confirming an ability to bind to human R/BBB (and brain antigen). According to this assay, plates coated with antigen (e.g. recombinant sR/BBB) are incubated with a sample comprising the monovalent binding entity towards the R/BBB and binding of the monovalent binding entity to the antigen of interest is determined.

In one aspect, the monovalent binding entity of the invention is tested for its antigen binding activity, e.g., by known methods such as ELISA, Western blot, etc.

In one aspect, the monovalent binding entity of the invention is tested for its single antigen binding activity towards an R/BBB using epitope mapping of X-ray structure determination.

Assays for evaluating uptake of systemically administered blood brain barrier shuttle and/or conjugate and other biological activity of blood brain barrier shuttle and/or conjugate can be performed as disclosed in the examples or as known for the blood brain barrier shuttle and/or conjugate of interest. Measuring the concentration within the parenchyma space of CNS can also be used using for example microdialysis or the capillary depletion method combined with ELISA or radioactivity measurements of labeled blood brain barrier shuttle and/or conjugate.

PHARMACEUTICAL FORMULATIONS

Therapeutic formulations of the blood brain barrier shuttle and/or conjugate used in accordance with the present invention are prepared for storage by mixing with optional pharmaceutically acceptable carriers, excipients or stabilizers {Remington 's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The formulation herein may also contain more than one active compound as necessary, optionally those with complementary activities that do not adversely affect each other. The type and effective amounts of such medicaments depend, for example, on the amount of blood brain barrier shuttle and/or conjugate present in the formulation, and clinical parameters of the subjects. Exemplary such medicaments are discussed below.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semi-permeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethylmethacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes. In one embodiment the formulation is isotonic.

The blood brain barrier shuttle and/or the conjugate of the invention may be utilized in a variety of in vivo methods. For example, the disclosure provides a method of transporting a therapeutic compound across the BBB comprising exposing the blood brain barrier shuttle and/or conjugate to the BBB such that the monovalent binding entity transports the therapeutic compound coupled thereto across the BBB. In another example, the disclosure provides a method of transporting a neurological disorder drug across the BBB comprising exposing the blood brain barrier shuttle and/or conjugate to the BBB such that the monovalent binding entity transports the neurological disorder drug coupled thereto across the BBB. In one embodiment, the BBB here is in a mammal (e.g. a human), e.g. one which has a neurological disorder, including, without limitation: Alzheimer's disease (AD), stroke, dementia, muscular dystrophy (MD), multiple sclerosis (MS), amyotrophic lateral sclerosis (ALS), cystic fibrosis, Angelman's syndrome, Liddle syndrome, Parkinson's disease, Pick's disease, Paget's disease, cancer, traumatic brain injury, etc.

In one embodiment, neurological disorder is selected from: a neuropathy, amyloidosis, cancer (e.g. involving the CNS or brain), an ocular disease or disorder, a viral or microbial infection, inflammation (e.g. of the CNS or brain), ischemia, neurodegenerative disease, seizure, behavioral disorder, lysosomal storage disease, etc.

Neuropathy disorders are diseases or abnormalities of the nervous system characterized by inappropriate or uncontrolled nerve signaling or lack thereof, and include, but are not limited to, chronic pain (including nociceptive pain), pain caused by an injury to body tissues, including cancer-related pain, neuropathic pain (pain caused by abnormalities in the nerves, spinal cord, or brain), and psychogenic pain (entirely or mostly related to a psychological disorder), headache, migraine, neuropathy, and symptoms and syndromes often accompanying such neuropathy disorders such as vertigo or nausea.

Amyloidoses are a group of diseases and disorders associated with extracellular proteinaceous deposits in the CNS, including, but not limited to, secondary amyloidosis, age- related amyloidosis, Alzheimer's Disease (AD), mild cognitive impairment (MCI), Lewy body dementia, Down's syndrome, hereditary cerebral hemorrhage with amyloidosis (Dutch type); the Guam Parkinson-Dementia complex, cerebral amyloid angiopathy, Huntington's disease, progressive supranuclear palsy, multiple sclerosis; Creutzfeld Jacob disease, Parkinson's disease, transmissible spongiform encephalopathy, HIV-related dementia, amyotropic lateral sclerosis (ALS), inclusion-body myositis (IBM), and ocular diseases relating to beta-amyloid deposition (i.e., macular degeneration, drusen-related optic neuropathy, and cataract).

Cancers of the CNS are characterized by aberrant proliferation of one or more CNS cell (i.e., a neural cell) and include, but are not limited to, glioma, glioblastoma multiforme, meningioma, astrocytoma, acoustic neuroma, chondroma, oligodendroglioma, meduUoblastomas, ganglioglioma, Schwannoma, neurofibroma, neuroblastoma, and extradural, intramedullary or intradural tumors.

Viral or microbial infections of the CNS include, but are not limited to, infections by viruses (i.e., influenza, HIV, poliovirus, rubella,), bacteria (i.e., Neisseria sp., Streptococcus sp., Pseudomonas sp., Proteus sp., E. coli, S. aureus, Pneumococcus sp., Meningococcus sp., Haemophilus sp., and Mycobacterium tuberculosis) and other microorganisms such as fungi (i.e., yeast, Cryptococcus neoformans), parasites (i.e., toxoplasma gondii) or amoebas resulting in CNS pathophysiologies including, but not limited to, meningitis, encephalitis, myelitis, vasculitis and abscess, which can be acute or chronic. Inflammation of the CNS is inflammation that is caused by an injury to the CNS, which can be a physical injury (i.e., due to accident, surgery, brain trauma, spinal cord injury, concussion) or an injury due to or related to one or more other diseases or disorders of the CNS (i.e., abscess, cancer, viral or microbial infection).

Ischemia of the CNS, as used herein, refers to a group of disorders relating to aberrant blood flow or vascular behavior in the brain or the causes therefor, and includes, but is not limited to: focal brain ischemia, global brain ischemia, stroke (i.e., subarachnoid hemorrhage and intracerebral hemorrhage), and aneurysm.

Neurodegenerative diseases are a group of diseases and disorders associated with neural cell loss of function or death in the CNS, and include, but are not limited to: adrenoleukodystrophy, Alexander's disease, Alper's disease, amyotrophic lateral sclerosis, ataxia telangiectasia, Batten disease, cockayne syndrome, corticobasal degeneration, degeneration caused by or associated with an amyloidosis, Friedreich's ataxia, frontotemporal lobar degeneration, Kennedy's disease, multiple system atrophy, multiple sclerosis, primary lateral sclerosis, progressive supranuclear palsy, spinal muscular atrophy, transverse myelitis, Refsum's disease, and spinocerebellar ataxia.

Seizure diseases and disorders of the CNS involve inappropriate and/or abnormal electrical conduction in the CNS, and include, but are not limited to: epilepsy (i.e., absence seizures, atonic seizures, benign Rolandic epilepsy, childhood absence, clonic seizures, complex partial seizures, frontal lobe epilepsy, febrile seizures, infantile spasms, juvenile myoclonic epilepsy, juvenile absence epilepsy, Lennox-Gastaut syndrome, Landau-Kleffner Syndrome, Dravet's syndrome, Otahara syndrome, West syndrome, myoclonic seizures, mitochondrial disorders, progressive myoclonic epilepsies, psychogenic seizures, reflex epilepsy, Rasmussen's Syndrome, simple partial seizures, secondarily generalized seizures, temporal lobe epilepsy, toniclonic seizures, tonic seizures, psychomotor seizures, limbic epilepsy, partialonset seizures, generalizedonset seizures, status epilepticus, abdominal epilepsy, akinetic seizures, autonomic seizures, massive bilateral myoclonus, catamenial epilepsy, drop seizures, emotional seizures, focal seizures, gelastic seizures, Jacksonian March, Lafora Disease, motor seizures, multifocal seizures, nocturnal seizures, photosensitive seizure, pseudo seizures, sensory seizures, subtle seizures, sylvan seizures, withdrawal seizures, and visual reflex seizures).

Behavioral disorders are disorders of the CNS characterized by aberrant behavior on the part of the afflicted subject and include, but are not limited to: sleep disorders (i.e., insomnia, parasomnias, night terrors, circadian rhythm sleep disorders, and narcolepsy), mood disorders (i.e., depression, suicidal depression, anxiety, chronic affective disorders, phobias, panic attacks, obsessive-compulsive disorder, attention deficit hyperactivity disorder (ADHD), attention deficit disorder (ADD), chronic fatigue syndrome, agoraphobia, post-traumatic stress disorder, bipolar disorder), eating disorders (i.e., anorexia or bulimia), psychoses, developmental behavioral disorders (i.e., autism, Rett's syndrome, Aspberger's syndrome), personality disorders and psychotic disorders (i.e., schizophrenia, delusional disorder, and the like).

Lysosomal storage disorders are metabolic disorders which are in some cases associated with the CNS or have CNS-specific symptoms; such disorders include, but are not limited to: Tay-Sachs disease, Gaucher's disease, Fabry disease, mucopolysaccharidosis (types I, II, III, IV, V, VI and VII), glycogen storage disease, GM1 -gangliosidosis, metachromatic leukodystrophy, Farber's disease, Canavan's leukodystrophy, and neuronal ceroid lipofuscinoses types 1 and 2, Niemann-Pick disease, Pompe disease, and Krabbe's disease.

In one aspect, the blood brain barrier shuttle and/or conjugate of the invention for use as a medicament is provided. In further aspects, the blood brain barrier shuttle and/or conjugate of the invention for use in treating a neurological disease or disorder is provided (e.g., Alzheimer's disease). In certain embodiments, the blood brain barrier shuttle and/or conjugate of the invention for use in a method of treatment is provided. In certain embodiments, the invention provides the blood brain barrier shuttle and/or conjugate of the invention for use in a method of treating an individual having a neurological disease or disorder comprising administering to the individual an effective amount of the blood brain barrier shuttle and/or conjugate of the invention. An "individual" according to any of the above embodiments is optionally a human.

The blood brain barrier shuttle and/or conjugate of the invention can be used either alone or in combination with other agents in a therapy. For instance, the blood brain barrier shuttle and/or conjugate of the invention may be co-administered with at least one additional therapeutic agent. In certain embodiments, an additional therapeutic agent is a therapeutic agent effective to treat the same or a different neurological disorder as the blood brain barrier shuttle and/or conjugate of the invention is being employed to treat. Exemplary additional therapeutic agents include, but are not limited to: the various neurological drugs described above, cholinesterase inhibitors (such as donepezil, galantamine, rovastigmine, and tacrine), NMDA receptor antagonists (such as memantine), amyloid beta peptide aggregation inhibitors, antioxidants, γ-secretase modulators, nerve growth factor (NGF) mimics or NGF gene therapy, PPARγ agonists, HMS-CoA reductase inhibitors (statins), ampakines, calcium channel blockers, GABA receptor antagonists, glycogen synthase kinase inhibitors, intravenous immunoglobulin, muscarinic receptor agonists, nicrotinic receptor modulators, active or passive amyloid beta peptide immunization, phosphodiesterase inhibitors, serotonin receptor antagonists and anti-amyloid beta peptide antibodies. In certain embodiments, the at least one additional therapeutic agent is selected for its ability to mitigate one or more side effects of the neurological drug.

Such combination therapies noted above encompass combined administration (where two or more therapeutic agents are included in the same or separate formulations), and separate administration, in which case, administration of the blood brain barrier shuttle and/or conjugate of the invention can occur prior to, simultaneously, and/or following, administration of the additional therapeutic agent and/or adjuvant. Blood brain barrier shuttles and/or conjugates of the invention can also be used in combination with other interventional therapies such as, but not limited to, radiation therapy, behavioral therapy, or other therapies known in the art and appropriate for the neurological disorder to be treated or prevented. The blood brain barrier shuttle and/or conjugate of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration.

Dosing can be by any suitable route, e.g. by injections, such as intravenous or subcutaneous injections, depending in part on whether the administration is brief or chronic. Various dosing schedules including but not limited to monovalent or multiple administrations over various time- points, bolus administration, and pulse infusion are contemplated herein.

Blood brain barrier shuttle and/or conjugates of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The blood brain barrier shuttle and/or conjugates of the invention need not be, but is optionally formulated with one or more agents currently used to prevent or treat the disorder in question. The effective amount of such other agents depends on the amount of blood brain barrier shuttle and/or conjugate present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of blood brain barrier shuttle and/or conjugate of the invention (when used alone or in combination with one or more other additional therapeutic agents) will depend on the type of disease to be treated, the type of blood brain barrier shuttle and/or conjugate, the severity and course of the disease, whether the antibody is administered for preventive or therapeutic purposes, previous therapy, the patient's clinical history and response to the blood brain barrier shuttle and/or conjugate, and the discretion of the attending physician. The blood brain barrier shuttle and/or conjugate is suitably administered to the patient at one time or over a series of treatments. Depending on the type and severity of the disease, about 1 μg/kg to 15 mg/kg (e.g. 0.1 mg/kg-10mg/kg) of blood brain barrier shuttle and/or conjugate can be an initial candidate dosage for administration to the patient, whether, for example, by one or more separate administrations, or by continuous inmultimeric. One typical daily dosage might range from about 1 $\mu$g/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. However, other dosage regimens may be useful. The progress of this therapy is easily monitored by conventional techniques and assays.

ARTICLES OF MANUFACTURE

In another aspect of the invention, an article of manufacture containing materials useful for the treatment and/or prevention of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a blood brain shuttle and/or conjugate of the invention. The label or package insert indicates that the composition is used for treating the condition of choice. Moreover, the article of manufacture may comprise (a) a first container with a composition contained therein, wherein the composition comprises a blood brain barrier shuttle and/or conjugate of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The article of manufacture optionally further comprises a package insert with instructions for treating a neurological disorder in a subject, wherein the instructions indicate that treatment with the blood brain barrier shuttle and/or conjugate as disclosed herein treats the neurological disorder, and optionally indicates that the blood brain barrier shuttle and/or conjugate has improved uptake across the BBB due to the monovalent binding mode to the R/BBB.

EXAMPLES

**Example 1: Generation of the expression plasmids**

Description of the basic/standard mammalian expression plasmid

Desired proteins were expressed by transient transfection of human embryonic kidney cells (HEK 293). For the expression of a desired gene/protein (e.g. antibody-Fab multimeric protein) a transcription unit comprising the following functional elements was used:

- the immediate early enhancer and promoter from the human cytomegalovirus (P-CMV) including intron A,

- a human heavy chain immunoglobulin 5'-untranslated region (5'UTR),

- a murine immunoglobulin heavy chain signal sequence (SS),

- a gene/protein to be expressed (e.g. full length antibody heavy chain), and

- the bovine growth hormone polyadenylation sequence (BGH pA).

Beside the expression unit/cassette including the desired gene to be expressed the basic/standard mammalian expression plasmid contains:

- an origin of replication from the vector pUC18 which allows replication of this plasmid in E. coli, and

• a beta-lactamase gene which confers ampicillin resistance in E. coli.

**Expression plasmids coding for the following antibody-scFab fusion polypeptides/proteins were constructed:**

*Tetravalent Mab31-scFab(8D3) (Fig. 1C) (Mab31 = human monoclonal antibody recognizing Abeta. INN of Mab 31 = Gantenerumab)*

Heavy chain (10132_pPM284_Mab31(IgG1)-(G$_4$S)$_4$-VL-Ck-(G$_4$S)$_6$-GG-VH-CH1) (Seq. Id. No. 1) :

Composition of the Mab31-scFab(8D3) heavy chain fusion protein:

• Mab31 human IgG1 heavy chain without C-terminal Lys

• Glycine Serine-linker

• Variable light chain domain (VL) variant (L596V and L598I) of the mouse 8D3 anti-transferrin antibody (Boado, R.J. Zhang, Y. Wang, Y and Pardridge, W.M., Biotechnology and Bioengineering (2009) 102, 1251-1258)

• Human C-kappa light chain

• GlycineSerine-linker

• Variable heavy chain domain (VH) of the mouse 8D3 anti-transferrin antibody (Boado, R.J. Zhang, Y. Wang, Y and Pardridge, W.M., Biotechnology and Bioengineering (2009) 102, 1251-1258)

• Human IgG1 CH3 heavy chain domain

Light chain (5170-VL-Mab31-BsmI-L2-Neo-BGHpA) (Seq. Id. No. 2)

Composition of the Mab31 light chain

- Mab31 human Ckappa light chain

*Trivalent Mab31-scFab(8D3) (Fig. 1B)*

Knob heavy chain (10134_pPM287_Mab31(IgG1)_knob_SS_-(G$_4$S)$_4$-VL-Ck-(G$_4$S)$_6$-GG-VH-CH1) (Seq. Id. No. 3)

Composition of the knob Mab31-scFab(8D3) heavy chain fusion protein

• Mab31 human IgG1 heavy chain without C-terminal Lys containing the CH3 knob mutation T366W and the S354C mutation for the formation of an additional disulfide bridge

• GlycineSerine-linker

• Variable light chain domain (VL) variant (L596V and L598I) of the mouse 8D3 anti-transferrin antibody (Boado, R.J. Zhang, Y. Wang, Y and Pardridge, W.M., Biotechnology and Bioengineering (2009) 102, 1251-1258)

• Human C-kappa light chain

• GlycineSerine-linker

- Variable heavy chain domain (VH) of the mouse 8D3 anti-transferrin antibody (Boado, R.J. Zhang, Y. Wang, Y and Pardridge, W.M., Biotechnology and Bioengineering (2009) 102, 1251-1258)

- Human IgG1 CH3 heavy chain domain

Hole heavy chain (10133_pPM286_Mab31(IgG1)_hole_SS) (Seq. Id. No. 4) Composition of the hole Mab31 heavy chain fusion protein

- Mab31 human IgG1 heavy chain containing the CH3 hole mutations T366S, Y407V and L368A and the Y349C mutation for the formation of an additional disulfide bridge

Light chain (5170-VL-Mab31-BsmI-L2-Neo-BGHpA) (Seq. Id. No. 2) Composition of the Mab31 light chain

- Mab31 human Ckappa light chain

**Example 2: Purification of single and double Mab31-scFab constructs sFab and dFab**

The antibody chains were generated by transient transfection of HEK293 cells (human embryonic kidney cell line 293-derived) cultivated in F17 Medium (Invitrogen Corp.). For transfection "293-Fectin" Transfection Reagent (Invitrogen) was used. The antibody chains were expressed from two (tetravalent Mab31-scFab(8D3)) or three (trivalent Mab31-scFab(8D3)) different plasmids, coding for the tetravalent Mab31-scFab(8D3) heavy chain and the Mab31 corresponding light chain, or the knob and hole trivalent Mab31-scFab(8D3) heavy chains and the Mab31 corresponding light chain, respectively. The two or three plasmids were used at an equimolar plasmid ratio upon transfection. Transfections were performed as specified in the manufacturer's instructions. Antibody fusion proteins-containing cell culture supernatants were harvested seven days after transfection. Supernatants were stored frozen until purification.

Proteins were purified from filtered cell culture supernatants. Supernatants were applied to a protein A Sepharose column (GE Healthcare) and washed with PBS pH 7.4. Elution of antibodies was achieved with 100 mM Citate buffer at pH 3.0 followed by immediate neutralization of the sample to pH6.5. After concentration aggregated protein and other byproducts were separated from monomeric antibodies by size exclusion chromatography (Superdex 200; GE Healthcare) in 20 mM histidine, 140 mM NaCl, pH 6.0. Every single fraction was analyzed on analytical SEC (TSK G3000SWXL) and on a chip-based capillary electrophoresis system (CE-SDS, LabChipGX, Caliper) for the quantification of incompletely assembled molecules and other byproducts. Monomeric antibody fractions without byproducts were pooled. After concentration using a MILLIPOREAmicon Ultra (30 molecular weight cut off) centrifugal concentrator the protein was stored at -80 °C. Analytical characterization of the endproduct was done by UV protein determination, CE-SDS, size-exclusion chromatography, mass spectrometry and also by endotoxin determination.

**Example 3: ELISA binding data of single scFab (sFab) and double** scFab (dFab) **constructs**

Binding of mAb31-8D3 constructs to mouse transferrin receptor (mTfR) was assessed by indirect ELISA. To this end, recombinant mTfR (extracellular domain; Sino Biological) was coated to Maxisorb microtiter plate (Nunc) at 1 μg/mL in PBS at 4°C overnight. After blocking in 1% Crotein-C/PBS (blocking buffer; Roche) for 1 h at RT and 4 washes with 0.1% Tween-20/PBS (wash buffer), mAb31-8D3 constructs were added to the wells at concentrations between 0.01 and 150 nM in blocking buffer and incubated for 1 h at RT. After 4 wash steps, constructs were detected by addition of anti-human-IgG-HRP (Jackson Immunoresearch) at 1:10,000 dilution in blocking buffer (1 RT), followed by 6 washes and incubation in TMB (Sigma). Absorbance was read out at 450 nm after stopping color development with 1 N HCl.

Fig. 3 shows that binding of the bivalent mAb31-8D3-dFab to mTfR is comparable to that of 8D3 IgG, while the monovalent construct mAb31-8D3- sFab shows a reduced affinity.

Functionality of mAb31 was confirmed by ELISA. Briefly, Abeta(1-40) was coated at 7 μg/mL in PBS onto Maxisorp plates for 3 days at 37°C to produce fibrillar Abeta, then dried for 3 h at RT. The plate was blocked with 1% Crotein C and 0.1% RSA in PBS (blocking buffer) for 1 h at RT, then washed once with wash buffer. mAb31 constructs were added at concentrations up to 100 nM in blocking buffer and incubated at 4°C overnight. After 4 wash steps, constructs were detected as indicated above.

Fig. 4 shows that both mAb31-8D3 constructs (sFab and dFab) bind with an affinity comparable to that of unmodified mAb31 to immobilized Abeta fibrils.

**Example 4: Only single scFab constructs cross the BBB and decorates plaques**

Brain sectioning and immunohistochemical staining:

Brains were prepared after PBS perfusion and sagittal cryo-sections were cut between lateral ~ 1.92 and 1.68 millimeter according to the brain atlas of Paxinos and Franklin. Brains were sectioned at a nominal thickness of 20 microns at -15°C using a Leica CM3050 S cryostat and placed onto precooled glass slides (Superfrost plus, Menzel, Germany). For each brain, three sections spaced 80 microns were deposited on the same slide.

Sections were rehydrated in PBS for 5 minutes followed by immersion with 100% acetone precooled to -20°C for 2 min. All further steps were done at room temperature. Slides with brain sections were washed with PBS, pH 7.4 and blocking of unspecific binding sites by sequential incubation in Ultra V block (LabVision) for 5 minutes followed by PBS wash and incubation in power block solution (BioGenex) with 2 % normal goat serum in PBS for 20 min. Slides were directly incubated with the secondary antibody, an affinity-purified goat anti-human IgG (heavy and light chain specific) conjugated to Alexa Fluor 555 dye (# A-21433, lot 54699A, Molecular Probes) at a concentration of 20 microg/ml in 2 % normal goat serum in PBS, pH 7.4 for 1 hour. After extensive washing with PBS, plaque localization was assessed by a double-labeling for Abeta plaques by incubation with BAP-2, a Roche in-house murine monoclonal antibody against Abeta conjugated to Alexa Fluor 488 dye at 0.5 microg /ml for 1 hour in PBS with power block solution (BioGenex) and 10 % normal sheep serum. After PBS washing, autofluorescence of lipofuscin was reduced by quenching through incubation in 4mM CuSO4 in 50 mM ammonium acetate, pH 5 for 30 minutes. After rinsing the slides with double-distilled water, slides were embedded with Confocal Matrix (Micro Tech Lab, Austria).

Confocal microscopy

Three images from each section of the brain of each PS2APP-mouse with plaque containing regions in the frontal cortex (region of the primary motor cortex) were taken. Images were recorded with a Leica TCS SP5 confocal system with a pinhole setting of 1 Airy.

Plaques immunolabelled with Alexa Fluor 488 dyes were captured in the same spectral conditions (a 488nm excitation and a 500-554nm band pass emission) with adjusted photomultiplier gain and offset (typically, 770 V and -0% respectively) at a 30% laser power.

Bound secondary Alexa Fluor 555 antibodies on the accessible surface of tissue sections were recorded at the 561 nm excitation laser line at a window ranging from 570 to 725 nm covering the emission wavelength range of the applied detection antibody. Instrument settings were kept constant for image acquisitions to allow comparative intensity measurements for tested human anti-Aβ antibodies; in particular, laser power, scanning speed, gain and offset. Laser power was set to 30% and settings for PMT gain were typically 850 V and a nominal offset of 0%. This enabled visualization of both faint and strongly stained plaques with the same setting. Acquisition frequency was at 400 Hz.

Confocal scans were recorded as single optical layers with a HCX PL APO 20x 0.7 IMM UV objective in water, at a 512 x 512-pixel resolution and an optical measuring depth in the vertical axis was interactively controlled to ensure imaging within the tissue section. Amyloid-plaques located in layers 2-5 of the frontal cortex were imaged and fluorescent intensities quantified.

Statistical analysis

Immunopositive regions were visualized as TIFF images and processed for quantification of fluorescence intensity and area (measured in pixels) with ImageJ version 1.45 (NIH). For quantification, background intensities of 5 were subtracted in every image and positive regions smaller than 5 square pixels were filtered out. Total fluorescence intensity of selected isosurfaces was determined as sum of intensities of single individual positive regions and the mean pixel intensity was calculated dividing the total intensity by the number of pixels analyzed.

Average and standard deviations values were calculated with Microsoft Excel (Redmond / WA, USA) from all measured isosurfaces obtained from nine pictures taken from three different sections for each animal. Statistical analysis was performed using the Student's t test for group comparison or a Mann-Whitney test.

10 mg/ml of mAb31 (construct of Fig1A), 13.3 mg/kg sFab-mAb31 1 (construct of Fig1B) and 16.7 mg/kg of dFAb-mAb31 (construct of Fig1C) was i.v. tail injected in mice and after 8 hours the brain was perfused with PBS. Sections was prepared as described above and stained with the goat anti-human IgG. For the mAb31 construct almost no specific signal was detected (Fig 4A). For the sFab-mAb31 extensive staining of both the plaque and capillaries was detected (Fig. 4B) while the dFab-mAb31 only staining of the capillaries was detected (Fig 4C). This cleary showed that a monovalent binding mode (sFab-mAb31) to the Transferrin receptor is much more efficient bring the construct through the brain endothelial cells at the BBB. The quantification of the bivalent binding molecule (dFab-mAb31) is shown in Fig 5. The data shows that there is not increase in plaque decoration for the dFab-mAb31 construct, there is only an increase in total intensity due to the capillary accumulation of the construct.

**Example 5: Quantification of brain exposures with a single scFab construct**

The experimental procedure is described in Example 4. Quantification of the sFab-mAb31 brain exposure is shown in Fig 6 using 10 mg/ml of mAb31 (construct of Fig1A) and 13.3 mg/kg sFab-mAb31 (construct of Fig1B). Already 8 hours after the injection of the sFab-mAb31 construct there is a massive uptake compare to mAb31 (about 55-fold increase). Similar data was obtained after 24 hours post dose using 25 mg/ml of mAb31 (construct of Fig1A) and 33.3 mg/kg sFab-mAb31 (construct of Fig1B). Fig 6 also shows the transient capillary staining of the sFab-mAb31 illustrates the targeting effect and the crossing of the BBB over time. All these data are highly significant as indicated in Fig 6.

Fig 7 shows data of the mAb31 (construct of Fig. 1A) and the sFab-mAb31 (construct of Fig. 1B) construct at a low dose. Again only the sFab-mAb31 construct is able to cross the brain endothelial cells and decorate the plaque in the brain. Maximal effect is already reached at 8 hours post dose. It is only at a higher dose (10 mg/kg) and relative long time (7 days) for the mAb31 construct that there is a trend for increase in the signal of binding to the Abeta plaques in the brain (Fig 7). All these data are highly significant as indicated in Fig 7.

**Example 6: Specific down-regulation of cell surface TfR by a double scFab construct**

Experimental details: bEnd3 cells cultured in a 6-well plate format. 2-3 days after confluence treated with dFab-mAb31, sFab-mAb31 or untreated ctr. for 24 hours. Then medium removed/aspirated and cells washed twice with ice cold PBS (-MgCl)(-CaCl) (Gibco 14190-094), 5ml/well. 1ml Trypsin/EDTA (Lonza CC-5012)/ well were added, incubated at 37°C for 15 minutes until all cells were detached. Stopped reaction with 1ml trypsin neutralizing solution (ice-cold) (Lonza CC-5002). 2ml of the Trypsin/EDTA + neutralization solution collected in a 50ml Falcon tube and kept on ice. Centrifugation of the cells at 4°C with 1400 rpm for 10 minutes. Pellets re-suspended in 50ml ice cold bEnd3 Medium (DMEM-12 (Gibco 31331) + 10%FBS). Centrifugation of the cells at 4°C with 1400rpm for 10 minutes. Pellet re-suspended in 3ml ice cold FACS-Buffer (BD 554656). Cell counts: a) sFab tube (2,5x105 cells/ml) viability: 47%, b) dFab tube (3.18x10$^5$ cells/ml) viability: 55%, c) ctr. tube (4.6x105 cells/ml) viability: 57%. FACS staining 1x10$^5$ cells/eppendorf tube distributed and centrifuged (4°C,10min,1500rpm). Supernatant aspirated; a) CD71-PE (clone R17217- IgG2a monoclonal) (santa cruz sc-52504) 20 microL of the antibody/pellet (staining volume 100microL) filled up to 100 microL with ice cold FACS-Buffer (BD 554656), b) CD31-APC (BD 551262) (rat anti mouse IgG2a (200 microg/ml)) 5 microg antibody/pellet (staining volume 100 microL) filled up to 100 microL with ice cold FACS-Buffer (BD 554656), c) 8D3-Alexa488 (1:50) (staining volume 100 microL) diluted in ice cold FACS-Buffer (BD 554656), d) Isotype ctr. for Alexa488, APC and PE (all from BD). Incubation in the dark at ice for 1 hour. Filled up to 1.5 ml with ice cold FACS-Buffer and centrifuged (4°C,10 min,1500rpm). Washed pellet twice with 1.5 ml ice cold FACS-Buffer and finally re-suspended pellet in 500 microL PBS. FACS measurement was performed using the instrument Guava Flow Cytometry. The data shows that the double (dFab) construct (Fig 8B) appears to down-regulate the Transferrin receptor on the cell surface. This is not detectable in this assay setup with the single (sFab) construct (Fig 8A) indicating that a monovalent binding mode has no direct effect on the cell trafficking and recycling that determine the amount of the Transferrin receptor at the cell surface on brain endothelial cells.

**Example 7: In vivo intracellular sorting of a single and double scFab construct**

APPswe/PS2 transgenic mice were injected i.v (tail injection) with the following constructs MAb31 (10mg/kg), sFab-MAb31 (13.3 mg/kg) or dFab-MAb31 (17.44mg/kg). The injected dose reflects the molecule size with MAb31 used as reference. 15 minutes or 8 hours after the injection, mice were euthanized with $CO_2$ and treated as followed. The right cardiac atrium of the heart was cut open so that blood and perfusion solution can flow out. The left cardiac ventricle was incised and a gavage probe #10 was shoved into the aorta. Approximately 20 ml of PBS were injected (~10 ml/min, room temperature) followed by 30 ml of 2% PFA in PBS. Brains were taken out and incubated for an additional 7h00 in the same perfusat. Vibratome was used to generate 100 microns brain free-floating sections that were used for immunofluorescence staining. Sections were first permeabilized and blocked using PBS-0.3% Triton X-100-10% donkey serum. Then, sections were incubated overnight with indicated primary antibodies diluted in PBS-5% donkey serum. Molecular probes secondary antibodies were used following manufacturer recommendations. Images were acquired using a Leica SP5 confocal microscope, Imaris software was used for image processing and 3D reconstruction.

These data illustrates the uptake of peripherally administered sFab-MAb31 and dFab-MAb31 by brain endothelial cells. MAb31, sFab-MAb31 and dFab-MAb31 were detected using a goat anti-human antibody coupled to Alexa 555. As shown in Fig 9, both sFab-MAb31 (Fig. 9A) and dFab-MAb31 (Fig. 9B) decorates the brain vasculature 15 min after injection with no difference in their distribution. 8h00 post-injection, sFab-MAb31 reaches the parenchyma and decorates amyloid plaques (Fig. 9C arrows) whereas dFab-MAb31 (Fig. 9D) stays within brain vasculature similarly to the 15min time point. No amyloid plaques in the parenchyma are detected with the dFab-MAb31.

Fig 10: To control the integrity of all constructs used in the study, staining of 18 months brain cryosections was done using MAb31 (Fig. 10A), sFab-MAb31 (Fig. 10B) or dFab-MAB31 (Fig. 10C). Results showed that all 3 constructs detected amyloid plaques in the brain of transgenic mice.

Fig 11-12: High resolution confocal microscopy shows that sFab (Fig. 11) and dFab-MAb31 (Fig. 12) do not decorate the luminal side of brain capillaries but are contained within vesicule-like structures crossing the luminal membrane of endothelial cells and within the endothelial cell cytosol. Arrows in Fig 11 and Fig 12 indicate vesicles containing sFab or dFab-MAb31 constructs on the abluminal side of endothelial cell nuclei. Altogether these data suggest that both sFab-MAb31 and dFab-MAb31 can enter endothelial cells but only sFab-MAb31 can cross the vasculature and reach amyloid plaques

The methods and compositions of the invention provide a way to drastically improve the part of the antibody that distributes into the CNS and thus more readily reach a therapeutic concentration in the CNS. The methods and compositions of the present invention are novel and significantly improve the efficiency of crossing through the different organelles within the BECs using an optimal and undisturbed intracellular route/sorting to reach the abluminal side.

## Example 8: Monovalent receptor binding mode crucial for crossing the BBB

The anti-Aβ monoclonal antibody mAb31 is a very specific and potent Aβ plaque binder providing us with a powerful readout to quantify target engagement within brain parenchyma. We used the PS2APP double transgenic amyloidosis model to investigate the amount of brain exposure of the two Brain Shuttle constructs compared to the mAb31 parent antibody. The three variants were injected intravenously at 10 mg/kg and the degree of brain exposure was determined by quantifying the amount of antibody present at plaques 8 hours post injection. For the dFab construct no significant increase in plaque decoration was detected compared to mAb31 (Fig. 13A). However, for the sFab construct there was a massive increase in plaque decoration in comparison with the parent mAb31 antibody. Target engagement at the amyloid plaques was improved more than 50-fold for the sFab construct based on fluorescence intensity quantification using a labeled secondary antibody. Whereas the sFab construct showed extensive plaque decoration (Fig. 13D), the dFab was only detectable in the microvessels (Fig. 13C) indicating that the dFab construct targets and enters brain microvessels but fails to escape at the abluminal side. We investigated the target engagement capacity of the sFab construct at a low dose of 2.66 mg/kg and prolonged in vivo exposure time up to 7 days. Maximal plaque decoration was reached within 8 hours, followed by persistent plaque binding over at least one week after a single injection (Fig. 13E).

In a previous study, the parent mAb31 had been shown to reach maximal plaque binding 7 days after injection. Quantification of the staining in microvessel structures indicated that the localization of the sFab construct was very transient at the BBB, illustrating the relatively rapid rate at which the construct crosses the barrier. The representative plaque staining images for the parent antibody mAb31 at 2 mg/kg 7 day post injection (Fig. 13F) and equimolar concentration for the sFab construct (Fig. 13G) illustrate the increase in plaque binding one achieves with the sFab brain shuttle construct. The sFab construct shows only a minor colocalization with the lysosomal compartment, which likely reflects normal constitutive trafficking of the TfR to the lysosome. Our in vitro studies also showed recycling and transcytosis of the sFab construct. Taken together, these findings suggest that the sFab construct does not interfere with the normal trafficking of the TfR. In contrast, the dFab construct shows strong colocalization with the lysosomal compartment but no transcytosis activity, neither in vitro nor in vivo.

## Example 9: Increased antibody delivery across BBB translates into enhanced in vivo potency

In the next set of experiments, we asked whether the significant increase in brain exposure using a monovalent binding mode improves in vivo potency of the anti-Aβ antibody in a long-term treatment study. We injected the sFab construct and the control parent antibody mAb31 weekly for three months. In a previous 5-months study, the therapeutic antibody mAb31 had been shown to reduce the plaque burden at 20 mg/kg. Based on the data shown in Figure 14 we selected two low doses to investigate if improved brain exposure would lead to enhanced in vivo potency. Target plaque binding at the end indicated that at both doses there was stronger target engagement with the sFab construct than the parent mAb31 antibody (Fig. 14 A - D). The degree of amyloidosis in the APPPS2 double transgenic mice was quantified at baseline, and following vehicle, low dose parent mAb31 and low dose sFab construct treatment. At these low doses no in vivo effect was detected with the parent monoclonal mAb31 (Fig. 14E), which was anticipated based on a previous long-term study over 5-months. In contrast, a significant reduction in plaque numbers both in cortex and hippocampus was observed with the 2.67 mg/kg low dose of the sFab construct. Even at the much lower dose of 0.53 mg/kg (Fig. 14E), a trend was seen in favor of the sFab construct especially in the cortex, although it did not reached statistical significance. A secondary analysis of plaque sizes revealed a more pronounced reduction of plaque numbers for small plaques, in agreement with the mode of action for mAb31. These data indicate that increased brain penetration, enabled by a monovalent mode of TfR binding, leads to a significant improvement in potency of a therapeutic antibody in a chronic animal model of Alzheimer's disease pathology.

**Example 10: Effector function of different antibody fusion proteins on TfR+ BaF3 cells in vitro (ADCC)**

Transferrin receptor expressing BaF3 cells (DSMZ, # CLPZ04004) (TfR+) were used as target cells for antibody-dependent cell toxicity (ADCC) experiments induced by different antibody-fusion molecules.

Briefly, $1x10^4$ BaF3 cells were seeded in round bottom 96-wells and optionally co-cultured with human NK92 effector cells (high affinity CD16 clone 7A2F3; Roche GlycArt) at an effector/target ratio of 3:1 in the presence or absence of antibody fusion proteins. After four hours incubation (37°C, 5%CO2), cytotoxicity was assessed as measured by the release of lactate dehydrogenase (LDH) from dead/dying cells. For this cells were centrifuged for 5 min at 250xg and 50μl supernatant was transferred to a flat bottom plate. 50μl LDH reaction mix (Roche LDH reaction mix, cat. no. 11644793001; Roche Diagnostics GmbH) was added and the reaction was incubated for 20 min at 37°C, 5% $CO_2$. Subsequently, the absorbance was measured at a Tecan Sunrise Reader at 492/620nm wavelength.

All samples were tested in triplicates and the results calculated based the following controls:

- Only target cells (+ medium)

- Maximal LDH release: target cells + 3% Triton-X

- Spontaneous release: target cells + NK cells (E:T of 3:1)

% specific ADCC/lysis was calculated by the following term:

$$\% \text{ spec. ADCC} = \frac{\text{Sample} - \text{spontaneous release}}{\text{Maximal release} - \text{spontaneous release}} \times 100$$

Fig. 15: Antibody fusion with TfR scFab fragments fused to the Fc C-terminus do not induce ADCC. NK92-mediated killing of BA/F3 mouse erythroleukemia cells was measured by quantifying LDH release. Only fusion constructs with the TfR-binding Fab moiety in the "conventional" "N-terminal to Fc" orientation induce significant ADCC, while the brain shuttle constructs in reverse orientation are silent. Constructs: 8D3-IgG (full length 8D3 IgG), OA - 8D3 (single heavy chain of 8D3 IgG), mAb31 (antibody of Fig. 1A), mAb31-8D3 -sFab (construct of Fig. 1B), mAb31-8D3-dFab (construct of Fig. 1C).

**Example 11: Epitope mapping of mTfR antibody 8D3**

The epitope mapping of monoclonal antibody 8D3 was carried out by means of a library of overlapping, immobilized peptide fragments (length: 15 amino acids, shift: 3 amino acids) corresponding to the sequence of the extracellular domain of murine Transferrin receptor 1 (90-763). For preparation of the peptide array the Intavis CelluSpots™ technology was employed. In this approach, peptides are synthesized with an automated synthesizer (Intavis MultiPep RS) on modified cellulose disks which are dissolved after synthesis. The solutions of the individual peptides that remain covalently linked to macromolecular cellulose are then spotted onto coated microscope slides. The CelluSpots™ synthesis was carried out stepwise utilizing 9-fluorenylmethoxycarbonyl (Fmoc) chemistry on amino-modified cellulose disks in a 384-well synthesis plate. In each coupling cycle, the corresponding amino acids were activated with a solution of DIC/HOBt in DMF. Between coupling steps, un-reacted amino groups were capped with a mixture of acetic anhydride, diisopropylethyl amine and 1-hydroxybenzotriazole. Upon completion of the synthesis, the cellulose disks were transferred to a 96-well plate and treated with a mixture of trifluoroacetic acid (TFA), dichloromethane, triisoproylsilane (TIS) and water for side chain deprotection. After removal of the cleavage solution, the cellulose bound peptides are dissolved with a mixture of TFA, TFMSA, TIS and water, precipitated with diisopropyl ether and re-suspended in DMSO. These peptide solutions were subsequently spotted onto Intavis Cel-luSpots™ slides using an Intavis slide spotting robot.

For epitope analysis, the prepared slides were washed with ethanol and then Tris-buffered saline (TBS; 50 mM Tris, 137 mM NaCl, 2.7 mM KCl, pH 8) before a blocking step was carried out for 16 h at 4°C with 5 mL 10x Western Blocking Reagent (Roche Applied Science), 2.5 g sucrose in TBS, 0.1% Tween 20. After washing (TBS + 0.1% Tween 20), the slides were incubated with a solution (1 μg/mL) of antibody 8D3 in TBS + 0.1% Tween 20 at ambient temperature for 2 h. After washing, the slides were incubated for detection with an anti-mouse secondary HRP-antibody (1:20000 in TBS-T) followed by incubation with chemiluminescence substrate luminol and visualized with a LumiImager (Roche Applied Science). ELISA-positive SPOTs were quantified and through assignment of the corresponding peptide sequences the antibody binding epitopes were identified.

Fig. 16: 8D3 binds to three distinct peptides in the extracellular domain of mouse transferrin receptor. Binding of antibody 8D3 to 15mer peptides overlapping by three amino acids was revealed by chemiluminescent detection of antibody incubated on a CelluSpot slide carrying immobilized mTfR peptides. Box: Peptides #373, 374 and 376 bound by 8D3.

Table 1: mTfR extracellular domain peptide sequences bound by 8D3 in peptide mapping experiment.

| Peptide ID | Peptide Sequence | Sequence Number |
|---|---|---|
| 373 | I-G-Q-N-M-V-T-I-V-Q-S-N-G-N-L | Seq. Id. No. 14 |
| 374 | N-M-V-T-I-V-Q-S-N-G-N-L-D-P-V | Seq. Id. No. 15 |
| 376 | Q-S-N-G-N-L-D-P-V-E-S-P-E-G-Y | Seq. Id. No. 16 |

Herein is described a group of biotherapeutic constructs against a blood brain barrier receptor, in particular the transferrin receptor (TfR), that can deliver therapeutics including antibodies, proteins, peptides and small molecules across the BBB at therapeutically relevant doses. Distribution of certain engineered biotherapeutic constructs changed from cerebrovascular space to parenchyma space within a few hours after injection, indicating that these particular constructs utilizing an optimal transport pathway through the BECs to allow significant amount of biotherapeutics to be transcytosed through BECs to reach the parenchyma. The degree of biotherapeutic constructs uptake into and distribution in the CNS was completely dependent on the monovalent binding mode to the blood brain barrier receptor, in particular TfR. When the TfR become dimerized by the binding of the biotherapeutic construct to the R/BBB no detectable level within the parenchyma space was detected. A single systemic dose of the single Fab anti-Abeta monoclonal construct engineered using the methodology of the invention not only resulted in significant antibody uptake in brain, but also dramatically increase the decoration of the anti-Abeta monoclonal binding to pathological amyloid plaques. However, using a double Fab binding construct against the R/BBB, no detectable levels within the CNS was detected. The facts and experiments depicted in this application illustrate key contributing mechanisms behind increasing uptake of a biotherapeutics (such as antibodies) into the CNS using a monovalent binding mode against an R/BBB. First, a dual (or multimeric) anti-R/BBB binding mode limit brain uptake by quickly down-regulate the R/BBB on the cell surface on the lumen side, thus reducing the total amount anti-R/BBB that can be taken up into the vasculature which is the first step in efficient BBB crossing. Secondly, a dual (or multimeric) anti-R/BBB binding mode induces a distinct miss-sorting intracellularly in the BECs that prevent the construct to reach the abluminal side. Strikingly, monovalent binding to the R/BBB improves brain uptake and distribution, with a complete shift observed in localization from the vasculature to the amyloid plaques within the CNS. Second, the engineered monovalent binding mode of the biotherapeutic constructs for the R/BBB is securing the recycling of the R/BBB to the lumen side to allow uptake of additional fusion polypeptide construct and transport to the abluminal side and into the parenchyma. Third, the monovalent binding mode biotherapeutic construct is engineered at the C-terminal end of the Fc part of an IgG which preserve the original format for a therapeutic monoclonal antibody which in most cases are critical for in vivo efficacy. This can also be accomplished by linking to other part of an IgG described within this application. This is advantageous because already developed IgG monoclonals with established preclinical and clinical efficacy can be incorporated in this transport system without compromising established function and efficacy. Furthermore, receptor mediated transport (RMT)-based monovalent targeting R/BBB technology opens the door for a wide range of potential therapeutics for CNS diseases. The invention provides methods of engineering BBB-penetrant therapeutics preserving existing IgGs formats with proven therapeutic activities that greatly improve transport across the BBB and CNS distribution of the therapeutic.

**Disclosed amino acid sequences**

| Amino acid sequence name | Sequence Identification Number (Seq. Id. No.) |
|---|---|
| Mab31 heavy chain - scFab (8D3) | 1 |
| Mab31 light chain | 2 |
| Knob Mab31 heavy chain - scFab (8D3) | 3 |
| Hole Mab31 heavy chain - scFab (8D3) | 4 |
| Mab 31 V$_H$ CDR1 | 5 |
| Mab 31 V$_H$ CDR2 | 6 |
| Mab 31 V$_H$ CDR3 | 7 |

(continued)

| Amino acid sequence name | Sequence Identification Number (Seq. Id. No.) |
|---|---|
| Mab 31 $V_L$ CDR1 | 8 |
| Mab 31 $V_L$ CDR 2 | 9 |
| Mab 31 $V_L$ CDR3 | 10 |
| Mab 31 $V_H$ | 11 |
| Mab 31 $V_L$ | 12 |
| Peptide linker $(G_4S)_6G_2$ | 13 |
| 8D3 epitope mapping peptide 373 | 14 |
| 8D3 epitope mapping peptide 374 | 15 |
| 8D3 epitope mapping peptide 376 | 16 |
| Peptide linker $(G_4S)_4$ | 17 |

**Claims**

1. A blood brain barrier shuttle comprising a brain effector entity, a linker and one monovalent binding entity which binds to a transferrin receptor, wherein the linker couples the effector entity to the monovalent binding entity which binds to the transferrin receptor, wherein the monovalent binding entity which binds to the transferrin receptor comprises one scFab directed to the transferrin receptor recognizing an epitope in the transferrin receptor comprised within the amino acid sequence of Seq. Id. No. 14, 15 or 16 and wherein the brain effector entity is selected from the group consisting of neurological disorder drugs, neurotrophic factors and antibodies directed to a brain target selected from the group consisting of β-secretase 1, Aβ, epidermal growth factor, epidermal growth factor receptor 2, Tau, phosphorylated Tau, apolipoprotein E4, alpha synuclein, oligomeric fragments of alpha synuclein, CD20, huntingtin, prion protein, leucine rich repeat kinase 2, parkin, presenilin 2, gamma secretase, death receptor 6, amyloid precursor protein, p75 neurotrophin receptor and caspase 6.

2. The blood brain barrier shuttle of claim 1, wherein the brain effector entity is selected from the group consisting of proteins, polypeptides and peptides.

3. The blood brain barrier shuttle of claim 2, wherein the monovalent binding entity which binds to the transferrin receptor comprising one scFab directed to the transferrin receptor recognizing an epitope in the transferrin receptor comprised within the amino acid sequence of Seq. Id. No. 14, 15 or 16 is coupled to the C-terminal end of the brain effector entity by the linker.

4. The blood brain barrier shuttle of claims 1 to 3, wherein the brain effector entity comprises a full length antibody directed to the brain target.

5. The blood brain barrier shuttle of claim 4, wherein the full length antibody is a full length IgG.

6. The blood brain barrier shuttle of claim 5 comprising the full length IgG antibody as brain effector entity, the linker and one scFab directed to the transferrin receptor recognizing an epitope in the transferrin receptor comprised within the amino acid sequence of Seq. Id. No. 14, 15 or 16, wherein the scFab is coupled by the linker to the C-terminal end of the Fc part of one of the heavy chains of the IgG antibody.

7. The blood brain barrier shuttle of claims 4 - 6, wherein the effector entity is a full length antibody directed to Aβ.

8. The blood brain barrier shuttle of claim 7, wherein the antibody directed to Aβ comprises (a) H-CDR1 comprising the amino acid sequence of Seq. Id. No. 5, (b) H-CDR2 comprising the amino acid sequence of Seq. Id. No. 6, (c) H-CDR3 comprising the amino acid sequence of Seq. Id. No. 7, (d) L-CDR1 comprising the amino acid sequence of Seq. Id. No. 8, (e) L-CDR2 comprising the amino acid sequence of Seq. Id. No. 9 and (f) L-CDR3 comprising the amino acid sequence of Seq. Id. No. 10.

9. The blood brain barrier shuttle of claim 8, wherein the antibody directed to Abeta comprises a $V_H$ domain comprising the amino acid sequence of Seq. Id. No. 11 and a $V_L$ domain comprising the amino acid sequence of Seq. Id. No. 12.

10. The blood brain barrier shuttle of claims 4 to 6, wherein the effector entity is a full length antibody directed to phosphorylated Tau.

11. The blood brain barrier shuttle of claims 4 to 6, wherein the effector entity is a full length antibody directed to alpha synuclein.

12. The blood brain barrier shuttle of claims 1 to 11, wherein the linker is a peptide linker.

13. The blood brain barrier shuttle of claim 12, wherein the peptide linker is an amino acid sequence with a length of at least 20 amino acids.

14. The blood brain barrier shuttle of claim 13, wherein the peptide linker is an amino acid sequence with a length of 25 to 50 amino acids.

15. The blood brain barrier shuttle of claims 1, wherein the monovalent binding entity which binds to the transferrin receptor comprises a CH2-CH3 Ig entity and one scFab directed to the transferrin receptor recognizing an epitope

44

in the transferrin receptor comprised within the amino acid sequence of Seq. Id. No. 14, 15 or 16, wherein the scFab is coupled to a C-terminal end of the CH2-CH3 Ig entity by a second linker.

16. The blood brain barrier shuttle of claim 15 comprising the brain effector entity, the linker, the CH2-CH3 Ig domain, the second linker and one scFab directed to the transferrin receptor recognizing an epitope in the transferrin receptor comprised within the amino acid sequence of Seq. Id. No. 14, 15 or 16, wherein the brain effector entity is coupled by the first linker to a N-terminal end of the CH2-CH3 Ig domain and the scFab is coupled to a C-terminal end of the CH2-CH3 Ig domain by the second linker.

17. The blood brain barrier shuttle of claims 15 or 16, wherein the CH2-CH3 Ig entity is a CH2-CH3 IgG entity.

18. A fusion protein to transport a brain effector entity across the blood brain barrier comprising a CH2-CH3 Ig entity, a linker and one scFab directed to the transferrin receptor recognizing an epitope in the transferrin receptor comprised within the amino acid sequence of Seq. Id. No. 14, 15 or 16, wherein the scFab is coupled to a C-terminal end of the CH2-CH3 Ig entity by the linker and wherein the brain effector entity is selected from the group consisting of neurological disorder drugs, neurotrophic factors and antibody fragments directed to a brain target selected from the group consisting of scFv, Fv, scFab, Fab, VHH, F(ab')$_2$ or peptides directed to a brain target and wherein the brain target is selected from the group consisting of β-secretase 1, Aβ, epidermal growth factor, epidermal growth factor receptor 2, Tau, phosphorylated Tau, apolipoprotein E4, alpha synuclein, oligomeric fragments of alpha synuclein, CD20, huntingtin, prion protein, leucine rich repeat kinase 2, parkin, presenilin 2, gamma secretase, death receptor 6, amyloid precursor protein, p75 neurotrophin receptor and caspase 6.

19. The fusion protein to transport the brain effector entity across the blood brain barrier of claim 18, wherein the linker is peptide linker.

20. The fusion protein of claims 18 or 19, wherein the CH2-CH3 Ig entity is a CH2-CH3 IgG entity.

21. A conjugate comprising a fusion protein to transport a brain effector entity across the blood brain barrier of claims 18 or 19 and the brain effector entity coupled to a N-terminal end of the CH2-CH3 Ig entity of the fusion protein by a linker.

22. The conjugate of claim 21, wherein the brain effector entity is selected from the group consisting of proteins, polypeptides and peptides.

23. The conjugate of claim 22, wherein a C-terminal end of the effector entity is coupled to the N-terminal end of the CH2-CH3 Ig entity by the linker.

**Patentansprüche**

1. Blut-Hirn-Schranken-Shuttle, umfassend eine Hirn-Effektor-Einheit, einen Linker und eine monovalente Bindungseinheit, die an einen Transferrinrezeptor bindet, wobei der Linker die Effektor-Einheit an die monovalente, an den Transferrinrezeptor bindende Bindungseinheit bindet, wobei die monovalente, an den Transferrinrezeptor bindende Bindungseinheit ein scFab umfasst, das gegen den Transferrinrezeptor gerichtet ist und ein Epitop in dem Transferrinrezeptor erkennt, das innerhalb der Aminosäuresequenz von SEQ ID NO: 14, 15 oder 16 enthalten ist, und wobei die Hirn-Effektor-Einheit ausgewählt ist aus der Gruppe bestehend aus Arzneimitteln für neurologische Störungen, neurotrophen Faktoren und Antikörpern, die gegen ein Hirn-Ziel gerichtet sind, ausgewählt aus der Gruppe bestehend aus β-Secretase 1, Aβ, epidermalem Wachstumsfaktor, epidermalem Wachstumsfaktorrezeptor 2, Tau, phosphoryliertem Tau, Apolipoprotein E4, alpha-Synuclein, oligomeren Fragmenten von alpha-Synuclein, CD20, Huntingtin, Prionenprotein, leucinreicher Repeat-Kinase 2, Parkin, Presenilin 2, gamma-Secretase, Todesrezeptor 6, Amyloidvorläuferprotein, p75-Neurotrophinrezeptor und Caspase 6.

2. Blut-Hirn-Schranken-Shuttle nach Anspruch 1, wobei die Hirn-Effektor-Einheit ausgewählt ist aus der Gruppe bestehend aus Proteinen, Polypeptiden und Peptiden.

3. Blut-Hirn-Schranken-Shuttle nach Anspruch 2, wobei die monovalente, an den Transferrinrezeptor bindende Bindungseinheit, die ein scFab umfasst, das gegen den Transferrinrezeptor gerichtet ist und ein Epitop in dem Transferrinrezeptor erkennt, das innerhalb der Aminosäuresequenz von SEQ ID NO: 14, 15 oder 16 enthalten ist, durch den Linker an das C-terminale Ende der Hirn-Effektor-Einheit gekoppelt ist.

4. Blut-Hirn-Schranken-Shuttle nach den Ansprüchen 1 bis 3, wobei die Hirn-Effektor-Einheit einen Volllängenantikörper umfasst, der gegen das Hirn-Ziel gerichtet ist.

5. Blut-Hirn-Schranken-Shuttle nach Anspruch 4, wobei der Volllängenantikörper ein Volllängen-IgG ist.

6. Blut-Hirn-Schranken-Shuttle nach Anspruch 5, umfassend den Volllängen-IgG-Antikörper als Hirn-Effektor-Einheit, den Linker und ein gegen den Transferrinrezeptor gerichtetes scFab, das ein Epitop in dem Transferrinrezeptor erkennt, das innerhalb der Aminosäuresequenz von SEQ ID NO: 14, 15 oder 16 enthalten ist, wobei das scFab durch den Linker an das C-terminale Ende des Fc-Teils von einer der schweren Ketten des IgG-Antikörpers gekoppelt ist.

7. Blut-Hirn-Schranken-Shuttle nach den Ansprüchen 4-6, wobei die Effektor-Einheit ein Volllängenantikörper ist, der gegen Aβ gerichtet ist.

8. Blut-Hirn-Schranken-Shuttle nach Anspruch 7, wobei der gegen Aβ gerichtete Antikörper Folgendes umfasst: (a) H-CDR1, umfassend die Aminosäuresequenz von SEQ ID NO: 5, (b) H-CDR2, umfassend die Aminosäuresequenz von SEQ ID NO: 6, (c) H-CDR3, umfassend die Aminosäuresequenz von SEQ ID NO: 7, (d) L-CDR1, umfassend die Aminosäuresequenz von SEQ ID NO: 8, (e) L-CDR2, umfassend die Aminosäuresequenz von SEQ ID NO: 9, und (f) L-CDR3, umfassend die Aminosäuresequenz von SEQ ID NO: 10.

9. Blut-Hirn-Schranken-Shuttle nach Anspruch 8, wobei der gegen Abeta gerichtete Antikörper eine $V_H$-Domäne, umfassend die Aminosäuresequenz von SEQ ID NO: 11, und eine $V_L$-Domäne, umfassend die Aminosäuresequenz von SEQ ID NO: 12, umfasst.

10. Blut-Hirn-Schranken-Shuttle nach den Ansprüchen 4 bis 6, wobei die Effektor-Einheit ein Volllängenantikörper ist, der gegen phosphoryliertes Tau gerichtet ist.

11. Blut-Hirn-Schranken-Shuttle nach den Ansprüchen 4 bis 6, wobei die Effektor-Einheit ein Volllängenantikörper ist, der gegen alpha-Synuclein gerichtet ist.

12. Blut-Hirn-Schranken-Shuttle nach den Ansprüchen 1 bis 11, wobei der Linker ein Peptid-Linker ist.

13. Blut-Hirn-Schranken-Shuttle nach Anspruch 12, wobei der Peptid-Linker eine Aminosäuresequenz mit einer Länge von mindestens 20 Aminosäuren ist.

14. Blut-Hirn-Schranken-Shuttle nach Anspruch 13, wobei der Peptid-Linker eine Aminosäuresequenz mit einer Länge von mindestens 25 bis 50 Aminosäuren ist.

15. Blut-Hirn-Schranken-Shuttle nach Anspruch 1, wobei die monovalente, an den Transferrinrezeptor bindende Bindungseinheit eine CH2-CH3-Ig-Einheit und ein scFab umfasst, das gegen den Transferrinrezeptor gerichtet ist und ein Epitop in dem Transferrinrezeptor erkennt, das innerhalb der Aminosäuresequenz von SEQ ID NO: 14, 15 oder 16 enthalten ist, wobei das scFab durch einen zweiten Linker an ein C-terminales Ende der CH2-CH3-Ig-Einheit gekoppelt ist.

16. Blut-Hirn-Schranken-Shuttle nach Anspruch 15, umfassend die Hirn-Effektor-Einheit, den Linker, die CH2-CH3-Ig-Domäne, den zweiten Linker und ein gegen den Transferrinrezeptor gerichtetes scFab, das ein Epitop in dem Transferrinrezeptor erkennt, das innerhalb der Aminosäuresequenz von SEQ ID NO: 14, 15 oder 16 enthalten ist, wobei die Hirn-Effektor-Einheit durch den ersten Linker an ein N-terminales Ende der CH2-CH3-Ig-Domäne gekoppelt ist und das scFab durch den zweiten Linker an ein C-terminales Ende der CH2-CH3-Ig-Einheit gekoppelt ist.

17. Blut-Hirn-Schranken-Shuttle nach Anspruch 15 oder 16, wobei die CH2-CH3-Ig-Einheit eine CH2-CH3-IgG-Einheit ist.

18. Fusionsprotein zum Transport einer Hirn-Effektor-Einheit durch die Blut-Hirn-Schranke, umfassend eine CH2-CH3-Ig-Einheit, einen Linker und ein gegen den Transferrinrezeptor gerichtetes scFab, das ein Epitop in dem Transferrinrezeptor erkennt, das innerhalb der Aminosäuresequenz von SEQ ID NO: 14, 15 oder 16 enthalten ist, wobei scFab durch den Linker an ein C-terminales Ende der CH2-CH3-Ig-Einheit gekoppelt ist und wobei die Hirn-Effektor-Einheit ausgewählt ist aus der Gruppe bestehend aus Arzneimitteln für neurologische Störungen, neurotrophen Faktoren und Antikörperfragmenten, die gegen ein Hirn-Ziel gerichtet sind, ausgewählt aus der Gruppe bestehend aus scFv, Fv, scFab, Fab, VHH, F(ab')₂ oder Peptiden, die gegen ein Hirn-Ziel gerichtet sind, und wobei das Hirn-Ziel ausgewählt ist aus der Gruppe bestehend aus β-Secretase 1, Aβ, epidermalem Wachstumsfaktor, epidermalem Wachstumsfaktorrezeptor 2, Tau, phosphoryliertem Tau, Apolipoprotein E4, alpha-Synuclein, oligomeren Fragmenten von alpha-Synuclein, CD20, Huntingtin, Prionenprotein, leucinreicher Repeat-Kinase 2, Parkin, Presenilin 2, gamma-Secretase, Todesrezeptor 6, Amyloidvorstufenprotein, p75-Neurotrophinrezeptor und Caspase 6.

**19.** Fusionsprotein zum Transport der Hirn-Effektor-Einheit durch die Blut-Hirn-Schranke nach Anspruch 18, wobei der Linker ein Peptid-Linker ist.

**20.** Fusionsprotein nach Anspruch 18 oder 19, wobei die CH2-CH3-Ig-Einheit eine CH2-CH3-IgG-Einheit ist.

**21.** Konjugat, umfassend ein Fusionsprotein zum Transport einer Hirn-Effektor-Einheit durch die Blut-Hirn-Schranke nach Anspruch 18 oder 19 und die Hirn-Effektor-Einheit, die durch einen Linker an ein N-terminales Ende der CH2-CH3-Ig-Einheit des Fusionsproteins gekoppelt ist.

**22.** Konjugat nach Anspruch 21, wobei die Hirn-Effektor-Einheit ausgewählt ist aus der Gruppe bestehend aus Proteinen, Polypeptiden und Peptiden.

**23.** Konjugat nach Anspruch 22, wobei das C-terminale Ende der Effektor-Einheit durch den Linker an das N-terminale Ende der CH2-CH3-Ig-Einheit gekoppelt ist.

## Revendications

**1.** Navette de la barrière hémato-encéphalique comprenant une entité effectrice cérébrale, un lieur et une entité de liaison monovalente qui se lie à un récepteur de la transferrine, dans laquelle le lieur couple l'entité effectrice à l'entité de liaison monovalente qui se lie au récepteur de la transferrine, dans laquelle l'entité de liaison monovalente qui se lie au récepteur de la transferrine comprend un fragment scFab dirigé contre le récepteur de la transferrine reconnaissant un épitope dans le récepteur de la transferrine compris dans la séquence d'acides aminés de SEQ ID NO. 14, 15 ou 16 et dans laquelle l'entité effectrice cérébrale est choisie dans le groupe constitué par les médicaments contre des troubles neurologiques, les facteurs neurotrophiques et les anticorps dirigés contre une cible cérébrale choisie dans le groupe constitué par la β-sécrétase 1, l'Aβ, le facteur de croissance épidermique, le récepteur du facteur de croissance épidermique 2, la Tau, la Tau phosphorylée, l'apolipoprotéine E4, l'alpha-synucléine, des fragments oligomères de l'alpha-synucléine, le CD20, l'huntingtine, la protéine du prion, la kinase à séquence répétée riche en leucine 2, la parkine, la préséniline 2, la gamme-sécrétase, le récepteur de mort 6, la protéine précurseur amyloïde, le récepteur des neurotrophines p75 et la caspase 6.

**2.** Navette de la barrière hémato-encéphalique selon la revendication 1, dans lequel l'entité effectrice cérébrale est choisie dans le groupe constitué par les protéines, les polypeptides et les peptides.

**3.** Navette de la barrière hémato-encéphalique selon la revendication 2, dans laquelle l'entité de liaison monovalente qui se lie au récepteur de la transferrine comprenant un fragment scFab dirigé contre le récepteur de la transferrine reconnaissant un épitope dans le récepteur de la transferrine compris dans la séquence d'acides aminés de SEQ ID NO. 14, 15 ou 16, est couplée à l'extrémité C-terminale de l'entité effectrice cérébrale par le lieur.

**4.** Navette de la barrière hémato-encéphalique selon les revendications 1 à 3, dans laquelle l'entité effectrice cérébrale comprend un anticorps pleine longueur dirigé contre la cible cérébrale.

**5.** Navette de la barrière hémato-encéphalique selon la revendication 4, dans laquelle l'anticorps pleine longueur est une IgG pleine longueur.

**6.** Navette de la barrière hémato-encéphalique selon la revendication 5, comprenant l'anticorps IgG pleine longueur comme entité effectrice cérébrale, le lieur et un fragment scFab dirigé contre le récepteur de la transferrine reconnaissant un épitope dans le récepteur de la transferrine compris dans la séquence d'acides aminés de SEQ ID NO. 14, 15 ou 16, dans laquelle le fragment scFab est couplé par le lieur à l'extrémité C-terminale de la partie Fc d'une des chaînes lourdes de l'anticorps IgG.

7. Navette de la barrière hémato-encéphalique selon les revendications 4 à 6, dans laquelle l'entité effectrice est un anticorps pleine longueur dirigé contre l'Aβ.

8. Navette de la barrière hémato-encéphalique selon la revendication 7, dans laquelle l'anticorps dirigé contre l'Aβ comprend (a) une H-CDR1 comprenant la séquence d'acides aminés de SEQ ID NO. 5, (b) une H-CDR2 comprenant la séquence d'acides aminés de SEQ ID NO. 6, (c) une H-CDR3 comprenant la séquence d'acides aminés de SEQ ID NO. 7, (d) une L-CDR1 comprenant la séquence d'acides aminés de SEQ ID NO. 8, (e) une L-CDR2 comprenant la séquence d'acides aminés de SEQ ID NO. 9 et (f) une L-CDR3 comprenant la séquence d'acides aminés de SEQ ID NO. 10.

9. Navette de la barrière hémato-encéphalique selon la revendication 8, dans laquelle l'anticorps dirigé contre l'Aβ comprend un domaine $V_H$ comprenant la séquence d'acides aminés de SEQ ID NO. 11 et un domaine $V_L$ comprenant la séquence d'acides aminés de SEQ ID NO. 12.

10. Navette de la barrière hémato-encéphalique selon les revendications 4 à 6, dans laquelle l'entité effectrice est un anticorps pleine longueur dirigé contre la Tau phosphorylée.

11. Navette de la barrière hémato-encéphalique selon les revendications 4 à 6, dans laquelle l'entité effectrice est un anticorps pleine longueur dirigé contre l'alpha-synucléine.

12. Navette de la barrière hémato-encéphalique selon les revendications 1 à 11, dans laquelle le lieur est un lieur peptidique.

13. Navette de la barrière hémato-encéphalique selon la revendication 12, dans laquelle le lieur peptidique est une séquence d'acides aminés ayant une longueur d'au moins 20 acides aminés.

14. Navette de la barrière hémato-encéphalique selon la revendication 13, dans laquelle le lieur peptidique est une séquence d'acides aminés ayant une longueur de 25 à 50 acides aminés.

15. Navette de la barrière hémato-encéphalique selon la revendication 1, dans laquelle l'entité de liaison monovalente qui se lie au récepteur de la transferrine comprend une entité d'Ig à CH2-CH3 et un fragment scFab dirigé contre le récepteur de la transferrine reconnaissant un épitope dans le récepteur de la transferrine compris dans la séquence d'acides aminés de SEQ ID NO. 14, 15 ou 16, dans laquelle le fragment scFab est couplé à une extrémité C-terminale de l'entité d'Ig à CH2-CH3 par un second lieur.

16. Navette de la barrière hémato-encéphalique selon la revendication 15, comprenant l'entité effectrice cérébrale, le lieur, le domaine d'Ig à CH2-CH3, le second lieur et un fragment scFab dirigé contre le récepteur de la transferrine reconnaissant un épitope dans le récepteur de la transferrine compris dans la séquence d'acides aminés de SEQ ID NO. 14, 15 ou 16, dans laquelle l'entité effectrice cérébrale est couplée par le premier lieur à une extrémité N-terminale du domaine d'Ig à CH2-CH3 et le fragment scFab est couplé à une extrémité C-terminale du domaine d'Ig à CH2-CH3 par le second lieur.

17. Navette de la barrière hémato-encéphalique selon la revendication 15 ou 16, dans laquelle l'entité d'Ig à CH2-CH3 est une entité d'IgG à CH2-CH3.

18. Protéine de fusion pour transporter une entité effectrice cérébrale à travers la barrière hémato-encéphalique comprenant une entité d'Ig à CH2-CH3, un lieur et un fragment scFab dirigé contre le récepteur de la transferrine reconnaissant un épitope dans le récepteur de la transferrine compris dans la séquence d'acides aminés de SEQ ID N° 14, 15 ou 16, dans laquelle le fragment scFab est couplé à une extrémité C-terminale de l'entité d'Ig à CH2-CH3 par le lieur et dans laquelle l'entité effectrice cérébrale est choisie dans le groupe constitué par les médicaments contre des troubles neurologiques, les facteurs neurotrophiques et les fragments d'anticorps dirigés contre une cible cérébrale choisie dans le groupe constitué par scFv, Fv, scFab, Fab, VHH, F(ab')$_2$ ou des peptides dirigés contre une cible cérébrale et dans laquelle la cible cérébrale est choisie dans le groupe constitué par la β-sécrétase 1, l'Aβ, le facteur de croissance épidermique, le récepteur du facteur de croissance épidermique 2, la Tau, la Tau phosphorylée, l'apolipoprotéine E4, l'alpha-synucléine, des fragments oligomères de l'alpha-synucléine, le CD20, l'huntingtine, la protéine du prion, la kinase à séquence répétée riche en leucine 2, la parkine, la préséniline 2, la gamme-sécrétase, le récepteur de mort 6, la protéine précurseur amyloïde, le récepteur des neurotrophines p75 et la caspase 6.

19. Protéine de fusion pour transporter l'entité effectrice cérébrale à travers la barrière hémato-encéphalique selon la revendication 18, dans laquelle le lieur est un lieur peptidique.

20. Protéine de fusion selon la revendication 18 ou 19, dans laquelle l'entité d'Ig à CH2-CH3 est une entité d'IgG à CH2-CH3.

21. Conjugué comprenant une protéine de fusion pour transporter une entité effectrice cérébrale à travers la barrière hémato-encéphalique selon la revendication 18 ou 19 et l'entité effectrice cérébrale couplée à une extrémité N-terminale de l'entité d'Ig à CH2-CH3 de la protéine de fusion par un lieur.

22. Conjugué selon la revendication 21, dans lequel l'entité effectrice cérébrale est choisie dans le groupe constitué par les protéines, les polypeptides et les peptides.

23. Conjugué selon la revendication 22, dans lequel une extrémité C-terminale de l'entité effectrice est couplée à l'extrémité N-terminale de l'entité d'Ig à CH2-CH3 par le lieur.

Fig. 1A          Fig. 1B          Fig. 1C

<Aβ>             <Aβ>             <Aβ>

                 <mTfR>  sFab     dFab
                                  <mTfR>

EP 2 890 712 B1

**Fig. 2**

Fig. 3

EP 2 890 712 B1

**Fig. 4A**　　　　　　**Fig. 4B**　　　　　　**Fig. 4C**

mAb31　　　　　　　　sFab-mAb31　　　　　　　dFab-mAb31

EP 2 890 712 B1

EP 2 890 712 B1

**Fig. 5**

# Quantification:
## 555 nm fluorescence intensity. 8h post dose

**Fig. 6**

**Fig. 7**

EP 2 890 712 B1

Fig. 8A

Fig. 8B

8D3 – A$_{488}$

Fig. 9A

Fig. 9B

Fig. 9C

Fig. 9D

Fig. 10A  Fig. 10B  Fig. 10C  Fig. 10D

MAb31  sFab-MAb31  dFab-MAb31  Control

Fig. 11

Fig. 12

Fig. 13A

Fig. 13B

Fig. 13C

Fig. 13D

Fig. 13E

EP 2 890 712 B1

Fig. 14D
Mid dose sFab

Fig. 14C
Low dose sFab

Fig. 13G
sFab

Fig. 14B
Mid dose mAb31

Fig. 13F
mAb31

Fig. 14A
Low dose mAb31

Fig. 14E

EP 2 890 712 B1

Fig. 15

**Fig. 16**

Peptides #373, 374 and 376

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012075037 A
- US 2005147613 A
- US 4816567 A
- US 5693780 A
- US 5591669 A
- US 5589369 A
- US 5545807 A
- US 5565332 A
- US 5573905 A
- US 5567610 A
- US 5229275 A
- US 20020004587 A1, Miller
- WO 0042072 A, Presta, L.
- WO 9951642 A, Iduosogie
- US 20030157108 A, Presta, L.
- US 20040093621 A
- WO 2003011878 A, Jean-Mairet
- US 6602684 B, Umana
- WO 199730087 A, Patel
- WO 199858964 A, Raju, S.
- WO 199922764 A, Raju, S.
- US 20050123546 A, Umana
- US 5208020 A
- US 20050142141 A
- US 3773919 A
- WO 12182181 A

### Non-patent literature cited in the description

- **FELGENHAUER.** *Klin. Wschr.,* 1974, vol. 52, 1158-1164
- **CARTER P. ; RIDGWAY J.B.B. ; PRESTA L.G.** *Immunotechnology,* February 1996, vol. 2 (1), 73-73
- **SCHNEIDER et al.** *Nature,* 1984, vol. 311, 675-678
- **HUST M. et al.** *BMC Biotechnol.,* 08 March 2007, vol. 7, 14
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **MORRISON et al.** *Proc. Natl. Acad. Sci. USA,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol,* 1992, vol. 2, 593-596
- **JAKOBOVITS et al.** *Proc. Natl Acad. Sci. USA,* 1993, vol. 90, 2551
- **JAKOBOVITS et al.** *Nature,* 1993, vol. 362, 255-258
- **BRUGGERMANN et al.** *Year in Immuno.,* 1993, vol. 7, 33
- **MCCAFFERTY et al.** *Nature,* 1990, vol. 348, 552-553
- **JOHNSON et al.** *Current Opinion in Structural Biology,* 1993, vol. 3, 564-571
- **GRIFFITH et al.** *EMBO J.,* 1993, vol. 12, 725-734
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **CHARI et al.** *Cancer Res.,* 1992, vol. 52, 127-131
- **ZATSEPIN et al.** *Russ. Chem. Rev.,* 2005, vol. 74, 77-95
- **FLATMAN et al.** *J. Chromatogr. B,* 2007, vol. 848, 79-87
- **AGNEW.** *Chem Intl. Ed. Engl,* 1994, vol. 33, 183-186
- **RATLIFF-SCHAUB et al.** *Autism,* 2005, vol. 9, 256-265
- **COWEN et al.** *J. Neurochem.,* 2004, vol. 89, 273-285
- **HETHWA et al.** *Am. J. Physiol.,* 2005, 301-305
- Remington's Pharmaceutical Sciences. 1980
- Remington's Pharmaceutical Sciences. 1980
- **BOADO, R.J. ; ZHANG, Y. ; WANG, Y ; PARDRIDGE, W.M.** Variable light chain domain (VL) variant (L596V and L598I) of the mouse 8D3 anti-transferrin antibody. *Biotechnology and Bioengineering,* 2009, vol. 102, 1251-1258

- **BOADO, R.J. ; ZHANG, Y. ; WANG, Y ; PARDRIDGE, W.M.** Variable heavy chain domain (VH) of the mouse 8D3 anti-transferrin antibody. *Biotechnology and Bioengineering,* 2009, vol. 102, 1251-1258

SEQUENCE LISTING

<110> F. Hoffmann-La Roche AG

<120> Blood brain barrier shuttles

<130> 30805 WO

<150> 12182181.3
<151> 2012-08-29

<160> 17

<170> PatentIn version 3.5

<210> 1
<211> 959
<212> PRT
<213> Artificial Sequence

<220>
<223> Fusion polypeptide

<400> 1

```
Met Gly Trp Ser Cys Ile Ile Leu Phe Leu Val Ala Thr Ala Thr Gly
1               5                   10              15

Val His Ser Gln Val Glu Leu Val Glu Ser Gly Gly Gly Leu Val Gln
            20                  25              30

Pro Gly Gly Ser Leu Arg Leu Ser Cys Ala Ala Ser Gly Phe Thr Phe
        35                  40              45

Ser Ser Tyr Ala Met Ser Trp Val Arg Gln Ala Pro Gly Lys Gly Leu
    50                  55                  60

Glu Trp Val Ser Ala Ile Asn Ala Ser Gly Thr Arg Thr Tyr Tyr Ala
65                  70                  75                  80

Asp Ser Val Lys Gly Arg Phe Thr Ile Ser Arg Asp Asn Ser Lys Asn
            85                  90                  95

Thr Leu Tyr Leu Gln Met Asn Ser Leu Arg Ala Glu Asp Thr Ala Val
        100                 105                 110

Tyr Tyr Cys Ala Arg Gly Lys Gly Asn Thr His Lys Pro Tyr Gly Tyr
        115                 120                 125

Val Arg Tyr Phe Asp Val Trp Gly Gln Gly Thr Leu Val Thr Val Ser
        130                 135                 140

Ser Ala Ser Thr Lys Gly Pro Ser Val Phe Pro Leu Ala Pro Ser Ser
145                 150                 155                 160
```

28

```
Lys Ser Thr Ser Gly Gly Thr Ala Ala Leu Gly Cys Leu Val Lys Asp
            165             170             175

Tyr Phe Pro Glu Pro Val Thr Val Ser Trp Asn Ser Gly Ala Leu Thr
            180             185             190

Ser Gly Val His Thr Phe Pro Ala Val Leu Gln Ser Ser Gly Leu Tyr
            195             200             205

Ser Leu Ser Ser Val Val Thr Val Pro Ser Ser Ser Leu Gly Thr Gln
    210             215             220

Thr Tyr Ile Cys Asn Val Asn His Lys Pro Ser Asn Thr Lys Val Asp
225             230             235             240

Lys Lys Val Glu Pro Lys Ser Cys Asp Lys Thr His Thr Cys Pro Pro
            245             250             255

Cys Pro Ala Pro Glu Leu Leu Gly Gly Pro Ser Val Phe Leu Phe Pro
            260             265             270

Pro Lys Pro Lys Asp Thr Leu Met Ile Ser Arg Thr Pro Glu Val Thr
            275             280             285

Cys Val Val Val Asp Val Ser His Glu Asp Pro Glu Val Lys Phe Asn
    290             295             300

Trp Tyr Val Asp Gly Val Glu Val His Asn Ala Lys Thr Lys Pro Arg
305             310             315             320

Glu Glu Gln Tyr Asn Ser Thr Tyr Arg Val Val Ser Val Leu Thr Val
            325             330             335

Leu His Gln Asp Trp Leu Asn Gly Lys Glu Tyr Lys Cys Lys Val Ser
            340             345             350

Asn Lys Ala Leu Pro Ala Pro Ile Glu Lys Thr Ile Ser Lys Ala Lys
            355             360             365

Gly Gln Pro Arg Glu Pro Gln Val Tyr Thr Leu Pro Pro Ser Arg Asp
            370             375             380

Glu Leu Thr Lys Asn Gln Val Ser Leu Thr Cys Leu Val Lys Gly Phe
385             390             395             400

Tyr Pro Ser Asp Ile Ala Val Glu Trp Glu Ser Asn Gly Gln Pro Glu
```

29

```
                        405                     410                         415


        Asn Asn Tyr Lys Thr Thr Pro Pro Val Leu Asp Ser Asp Gly Ser Phe
                    420                     425                     430


        Phe Leu Tyr Ser Lys Leu Thr Val Asp Lys Ser Arg Trp Gln Gln Gly
                    435                     440                     445


        Asn Val Phe Ser Cys Ser Val Met His Glu Ala Leu His Asn His Tyr
                    450                     455                     460


        Thr Gln Lys Ser Leu Ser Leu Ser Pro Gly Gly Gly Ser Gly Gly Gly
        465                     470                     475                     480


        Gly Ser Gly Gly Gly Gly Ser Gly Gly Gly Gly Ser Asp Ile Gln Met
                            485                     490                     495


        Thr Gln Ser Pro Ala Ser Leu Ser Ala Ser Leu Glu Glu Ile Val Thr
                    500                     505                     510


        Ile Thr Cys Gln Ala Ser Gln Asp Ile Gly Asn Trp Leu Ala Trp Tyr
                    515                     520                     525


        Gln Gln Lys Pro Gly Lys Ser Pro Gln Leu Leu Ile Tyr Gly Ala Thr
                    530                     535                     540


        Ser Leu Ala Asp Gly Val Pro Ser Arg Phe Ser Gly Ser Arg Ser Gly
        545                     550                     555                     560


        Thr Gln Phe Ser Leu Lys Ile Ser Arg Val Gln Val Glu Asp Ile Gly
                    565                     570                     575


        Ile Tyr Tyr Cys Leu Gln Ala Tyr Asn Thr Pro Trp Thr Phe Gly Gly
                    580                     585                     590


        Gly Thr Lys Val Glu Ile Lys Arg Thr Val Ala Ala Pro Ser Val Phe
                    595                     600                     605


        Ile Phe Pro Pro Ser Asp Glu Gln Leu Lys Ser Gly Thr Ala Ser Val
                    610                     615                     620


        Val Cys Leu Leu Asn Asn Phe Tyr Pro Arg Glu Ala Lys Val Gln Trp
        625                     630                     635                     640


        Lys Val Asp Asn Ala Leu Gln Ser Gly Asn Ser Gln Glu Ser Val Thr
                    645                     650                     655
```

```
    Glu Gln Asp Ser Lys Asp Ser Thr Tyr Ser Leu Ser Ser Thr Leu Thr
                660             665             670

    Leu Ser Lys Ala Asp Tyr Glu Lys His Lys Val Tyr Ala Cys Glu Val
                675             680             685

    Thr His Gln Gly Leu Ser Ser Pro Val Thr Lys Ser Phe Asn Arg Gly
                690             695             700

    Glu Cys Gly Gly Gly Gly Ser Gly Gly Gly Gly Ser Gly Gly Gly Gly
    705             710             715             720

    Ser Gly Gly Gly Gly Ser Gly Gly Gly Gly Ser Gly Gly Gly Gly Ser
                725             730             735

    Gly Gly Glu Val Gln Leu Val Glu Ser Gly Gly Gly Leu Val Gln Pro
                740             745             750

    Gly Asn Ser Leu Thr Leu Ser Cys Val Ala Ser Gly Phe Thr Phe Ser
                755             760             765

    Asn Tyr Gly Met His Trp Ile Arg Gln Ala Pro Lys Lys Gly Leu Glu
                770             775             780

    Trp Ile Ala Met Ile Tyr Tyr Asp Ser Ser Lys Met Asn Tyr Ala Asp
    785             790             795             800

    Thr Val Lys Gly Arg Phe Thr Ile Ser Arg Asp Asn Ser Lys Asn Thr
                805             810             815

    Leu Tyr Leu Glu Met Asn Ser Leu Arg Ser Glu Asp Thr Ala Met Tyr
                820             825             830

    Tyr Cys Ala Val Pro Thr Ser His Tyr Val Val Asp Val Trp Gly Gln
                835             840             845

    Gly Val Ser Val Thr Val Ser Ser Ala Ser Thr Lys Gly Pro Ser Val
                850             855             860

    Phe Pro Leu Ala Pro Ser Ser Lys Ser Thr Ser Gly Gly Thr Ala Ala
    865             870             875             880

    Leu Gly Cys Leu Val Lys Asp Tyr Phe Pro Glu Pro Val Thr Val Ser
                885             890             895

    Trp Asn Ser Gly Ala Leu Thr Ser Gly Val His Thr Phe Pro Ala Val
                900             905             910
```

31

```
Leu Gln Ser Ser Gly Leu Tyr Ser Leu Ser Ser Val Val Thr Val Pro
        915             920             925

Ser Ser Ser Leu Gly Thr Gln Thr Tyr Ile Cys Asn Val Asn His Lys
        930             935             940

Pro Ser Asn Thr Lys Val Asp Lys Lys Val Glu Pro Lys Ser Cys
945             950             955
```

<210> 2
<211> 234
<212> PRT
<213> Artificial Sequence

<220>
<223> Light chain construct

<400> 2

```
Met Gly Trp Ser Cys Ile Ile Leu Phe Leu Val Ala Thr Ala Thr Gly
1           5               10              15

Val His Ser Asp Ile Val Leu Thr Gln Ser Pro Ala Thr Leu Ser Leu
        20              25              30

Ser Pro Gly Glu Arg Ala Thr Leu Ser Cys Arg Ala Ser Gln Ser Val
        35              40              45

Ser Ser Ser Tyr Leu Ala Trp Tyr Gln Gln Lys Pro Gly Gln Ala Pro
        50              55              60

Arg Leu Leu Ile Tyr Gly Ala Ser Ser Arg Ala Thr Gly Val Pro Ala
65              70              75              80

Arg Phe Ser Gly Ser Gly Ser Gly Thr Asp Phe Thr Leu Thr Ile Ser
            85              90              95

Ser Leu Glu Pro Glu Asp Phe Ala Thr Tyr Tyr Cys Leu Gln Ile Tyr
            100             105             110

Asn Met Pro Ile Thr Phe Gly Gln Gly Thr Lys Val Glu Ile Lys Arg
        115             120             125

Thr Val Ala Ala Pro Ser Val Phe Ile Phe Pro Pro Ser Asp Glu Gln
        130             135             140

Leu Lys Ser Gly Thr Ala Ser Val Val Cys Leu Leu Asn Asn Phe Tyr
145             150             155             160
```

```
Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
            195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230
```

<210> 3
<211> 959
<212> PRT
<213> Artificial Sequence

<220>
<223> Fusion polypeptide

<400> 3

```
Met Gly Trp Ser Cys Ile Ile Leu Phe Leu Val Ala Thr Ala Thr Gly
1               5                   10                  15

Val His Ser Gln Val Glu Leu Val Glu Ser Gly Gly Gly Leu Val Gln
            20                  25                  30

Pro Gly Gly Ser Leu Arg Leu Ser Cys Ala Ala Ser Gly Phe Thr Phe
            35                  40                  45

Ser Ser Tyr Ala Met Ser Trp Val Arg Gln Ala Pro Gly Lys Gly Leu
    50                  55                  60

Glu Trp Val Ser Ala Ile Asn Ala Ser Gly Thr Arg Thr Tyr Tyr Ala
65                  70                  75                  80

Asp Ser Val Lys Gly Arg Phe Thr Ile Ser Arg Asp Asn Ser Lys Asn
                85                  90                  95

Thr Leu Tyr Leu Gln Met Asn Ser Leu Arg Ala Glu Asp Thr Ala Val
            100                 105                 110

Tyr Tyr Cys Ala Arg Gly Lys Gly Asn Thr His Lys Pro Tyr Gly Tyr
        115                 120                 125

Val Arg Tyr Phe Asp Val Trp Gly Gln Gly Thr Leu Val Thr Val Ser
```

                130                         135                              140

Ser Ala Ser Thr Lys Gly Pro Ser Val Phe Pro Leu Ala Pro Ser Ser
145                 150                 155                 160

Lys Ser Thr Ser Gly Gly Thr Ala Ala Leu Gly Cys Leu Val Lys Asp
                165                 170                 175

Tyr Phe Pro Glu Pro Val Thr Val Ser Trp Asn Ser Gly Ala Leu Thr
            180                 185                 190

Ser Gly Val His Thr Phe Pro Ala Val Leu Gln Ser Ser Gly Leu Tyr
            195                 200                 205

Ser Leu Ser Ser Val Val Thr Val Pro Ser Ser Ser Leu Gly Thr Gln
    210                 215                 220

Thr Tyr Ile Cys Asn Val Asn His Lys Pro Ser Asn Thr Lys Val Asp
225                 230                 235                 240

Lys Lys Val Glu Pro Lys Ser Cys Asp Lys Thr His Thr Cys Pro Pro
                245                 250                 255

Cys Pro Ala Pro Glu Leu Leu Gly Gly Pro Ser Val Phe Leu Phe Pro
            260                 265                 270

Pro Lys Pro Lys Asp Thr Leu Met Ile Ser Arg Thr Pro Glu Val Thr
            275                 280                 285

Cys Val Val Val Asp Val Ser His Glu Asp Pro Glu Val Lys Phe Asn
    290                 295                 300

Trp Tyr Val Asp Gly Val Glu Val His Asn Ala Lys Thr Lys Pro Arg
305                 310                 315                 320

Glu Glu Gln Tyr Asn Ser Thr Tyr Arg Val Val Ser Val Leu Thr Val
            325                 330                 335

Leu His Gln Asp Trp Leu Asn Gly Lys Glu Tyr Lys Cys Lys Val Ser
            340                 345                 350

Asn Lys Ala Leu Pro Ala Pro Ile Glu Lys Thr Ile Ser Lys Ala Lys
            355                 360                 365

Gly Gln Pro Arg Glu Pro Gln Val Tyr Thr Leu Pro Pro Cys Arg Asp
            370                 375                 380

```
Glu Leu Thr Lys Asn Gln Val Ser Leu Trp Cys Leu Val Lys Gly Phe
385             390             395                 400

Tyr Pro Ser Asp Ile Ala Val Glu Trp Glu Ser Asn Gly Gln Pro Glu
                405             410                 415

Asn Asn Tyr Lys Thr Thr Pro Pro Val Leu Asp Ser Asp Gly Ser Phe
            420             425             430

Phe Leu Tyr Ser Lys Leu Thr Val Asp Lys Ser Arg Trp Gln Gln Gly
            435             440             445

Asn Val Phe Ser Cys Ser Val Met His Glu Ala Leu His Asn His Tyr
450             455             460

Thr Gln Lys Ser Leu Ser Leu Ser Pro Gly Gly Gly Ser Gly Gly Gly
465             470             475                 480

Gly Ser Gly Gly Gly Gly Ser Gly Gly Gly Gly Ser Asp Ile Gln Met
            485             490             495

Thr Gln Ser Pro Ala Ser Leu Ser Ala Ser Leu Glu Glu Ile Val Thr
            500             505             510

Ile Thr Cys Gln Ala Ser Gln Asp Ile Gly Asn Trp Leu Ala Trp Tyr
        515             520             525

Gln Gln Lys Pro Gly Lys Ser Pro Gln Leu Leu Ile Tyr Gly Ala Thr
        530             535             540

Ser Leu Ala Asp Gly Val Pro Ser Arg Phe Ser Gly Ser Arg Ser Gly
545             550             555                 560

Thr Gln Phe Ser Leu Lys Ile Ser Arg Val Gln Val Glu Asp Ile Gly
            565             570             575

Ile Tyr Tyr Cys Leu Gln Ala Tyr Asn Thr Pro Trp Thr Phe Gly Gly
        580             585             590

Gly Thr Lys Val Glu Ile Lys Arg Thr Val Ala Ala Pro Ser Val Phe
        595             600             605

Ile Phe Pro Pro Ser Asp Glu Gln Leu Lys Ser Gly Thr Ala Ser Val
        610             615             620

Val Cys Leu Leu Asn Asn Phe Tyr Pro Arg Glu Ala Lys Val Gln Trp
625             630             635                 640
```

36

```
Lys Val Asp Asn Ala Leu Gln Ser Gly Asn Ser Gln Glu Ser Val Thr
            645             650             655

Glu Gln Asp Ser Lys Asp Ser Thr Tyr Ser Leu Ser Ser Thr Leu Thr
            660             665             670

Leu Ser Lys Ala Asp Tyr Glu Lys His Lys Val Tyr Ala Cys Glu Val
            675             680             685

Thr His Gln Gly Leu Ser Ser Pro Val Thr Lys Ser Phe Asn Arg Gly
    690             695             700

Glu Cys Gly Gly Gly Gly Ser Gly Gly Gly Ser Gly Gly Gly Gly
705             710             715             720

Ser Gly Gly Gly Gly Ser Gly Gly Gly Gly Ser Gly Gly Gly Gly Ser
            725             730             735

Gly Gly Glu Val Gln Leu Val Glu Ser Gly Gly Gly Leu Val Gln Pro
        740             745             750

Gly Asn Ser Leu Thr Leu Ser Cys Val Ala Ser Gly Phe Thr Phe Ser
        755             760             765

Asn Tyr Gly Met His Trp Ile Arg Gln Ala Pro Lys Lys Gly Leu Glu
    770             775             780

Trp Ile Ala Met Ile Tyr Tyr Asp Ser Ser Lys Met Asn Tyr Ala Asp
785             790             795             800

Thr Val Lys Gly Arg Phe Thr Ile Ser Arg Asp Asn Ser Lys Asn Thr
            805             810             815

Leu Tyr Leu Glu Met Asn Ser Leu Arg Ser Glu Asp Thr Ala Met Tyr
            820             825             830

Tyr Cys Ala Val Pro Thr Ser His Tyr Val Val Asp Val Trp Gly Gln
            835             840             845

Gly Val Ser Val Thr Val Ser Ser Ala Ser Thr Lys Gly Pro Ser Val
    850             855             860

Phe Pro Leu Ala Pro Ser Ser Lys Ser Thr Ser Gly Gly Thr Ala Ala
865             870             875             880

Leu Gly Cys Leu Val Lys Asp Tyr Phe Pro Glu Pro Val Thr Val Ser
            885             890             895
```

```
Trp Asn Ser Gly Ala Leu Thr Ser Gly Val His Thr Phe Pro Ala Val
            900             905             910

Leu Gln Ser Ser Gly Leu Tyr Ser Leu Ser Ser Val Val Thr Val Pro
            915             920             925

Ser Ser Ser Leu Gly Thr Gln Thr Tyr Ile Cys Asn Val Asn His Lys
            930             935             940

Pro Ser Asn Thr Lys Val Asp Lys Lys Val Glu Pro Lys Ser Cys
945             950             955
```

<210> 4
<211> 475
<212> PRT
<213> Artificial Sequence

<220>
<223> Fusion polypeptide

<400> 4

```
Met Gly Trp Ser Cys Ile Ile Leu Phe Leu Val Ala Thr Ala Thr Gly
1               5               10              15

Val His Ser Gln Val Glu Leu Val Glu Ser Gly Gly Gly Leu Val Gln
            20              25              30

Pro Gly Gly Ser Leu Arg Leu Ser Cys Ala Ala Ser Gly Phe Thr Phe
            35              40              45

Ser Ser Tyr Ala Met Ser Trp Val Arg Gln Ala Pro Gly Lys Gly Leu
            50              55              60

Glu Trp Val Ser Ala Ile Asn Ala Ser Gly Thr Arg Thr Tyr Tyr Ala
65              70              75              80

Asp Ser Val Lys Gly Arg Phe Thr Ile Ser Arg Asp Asn Ser Lys Asn
                85              90              95

Thr Leu Tyr Leu Gln Met Asn Ser Leu Arg Ala Glu Asp Thr Ala Val
            100             105             110

Tyr Tyr Cys Ala Arg Gly Lys Gly Asn Thr His Lys Pro Tyr Gly Tyr
            115             120             125

Val Arg Tyr Phe Asp Val Trp Gly Gln Gly Thr Leu Val Thr Val Ser
            130             135             140
```

```
Ser Ala Ser Thr Lys Gly Pro Ser Val Phe Pro Leu Ala Pro Ser Ser
145             150             155             160

Lys Ser Thr Ser Gly Gly Thr Ala Ala Leu Gly Cys Leu Val Lys Asp
                165             170             175

Tyr Phe Pro Glu Pro Val Thr Val Ser Trp Asn Ser Gly Ala Leu Thr
            180             185             190

Ser Gly Val His Thr Phe Pro Ala Val Leu Gln Ser Ser Gly Leu Tyr
            195             200             205

Ser Leu Ser Ser Val Val Thr Val Pro Ser Ser Ser Leu Gly Thr Gln
    210             215             220

Thr Tyr Ile Cys Asn Val Asn His Lys Pro Ser Asn Thr Lys Val Asp
225             230             235             240

Lys Lys Val Glu Pro Lys Ser Cys Asp Lys Thr His Thr Cys Pro Pro
                245             250             255

Cys Pro Ala Pro Glu Leu Leu Gly Gly Pro Ser Val Phe Leu Phe Pro
            260             265             270

Pro Lys Pro Lys Asp Thr Leu Met Ile Ser Arg Thr Pro Glu Val Thr
        275             280             285

Cys Val Val Val Asp Val Ser His Glu Asp Pro Glu Val Lys Phe Asn
    290             295             300

Trp Tyr Val Asp Gly Val Glu Val His Asn Ala Lys Thr Lys Pro Arg
305             310             315             320

Glu Glu Gln Tyr Asn Ser Thr Tyr Arg Val Val Ser Val Leu Thr Val
                325             330             335

Leu His Gln Asp Trp Leu Asn Gly Lys Glu Tyr Lys Cys Lys Val Ser
            340             345             350

Asn Lys Ala Leu Pro Ala Pro Ile Glu Lys Thr Ile Ser Lys Ala Lys
        355             360             365

Gly Gln Pro Arg Glu Pro Gln Val Cys Thr Leu Pro Pro Ser Arg Asp
    370             375             380

Glu Leu Thr Lys Asn Gln Val Ser Leu Ser Cys Ala Val Lys Gly Phe
385             390             395             400
```

```
Tyr Pro Ser Asp Ile Ala Val Glu Trp Glu Ser Asn Gly Gln Pro Glu
            405                 410                 415

Asn Asn Tyr Lys Thr Thr Pro Pro Val Leu Asp Ser Asp Gly Ser Phe
            420                 425                 430

Phe Leu Val Ser Lys Leu Thr Val Asp Lys Ser Arg Trp Gln Gln Gly
            435                 440                 445

Asn Val Phe Ser Cys Ser Val Met His Glu Ala Leu His Asn His Tyr
            450                 455                 460

Thr Gln Lys Ser Leu Ser Leu Ser Pro Gly Lys
465                 470                 475
```

<210> 5
<211> 10
<212> PRT
<213> Homo sapiens

<400> 5

```
            Gly Phe Thr Phe Ser Ser Tyr Ala Met Ser
            1               5               10
```

<210> 6
<211> 17
<212> PRT
<213> Homo sapiens

<400> 6

```
            Ala Ile Asn Ala Ser Gly Thr Arg Thr Tyr Tyr Ala Asp Ser Val Lys
            1               5               10                  15

            Gly
```

<210> 7
<211> 17
<212> PRT
<213> Homo sapiens

<400> 7

```
            Gly Lys Gly Asn Thr His Lys Pro Tyr Gly Tyr Val Arg Tyr Phe Asp
            1               5               10                  15

            Val
```

<210> 8
<211> 12
<212> PRT
<213> Homo sapiens

<400> 8

```
Arg Ala Ser Gln Ser Val Ser Ser Ser Tyr Leu Ala
1               5                   10
```

<210> 9
<211> 7
<212> PRT
<213> Homo sapiens

<400> 9

```
Gly Ala Ser Ser Arg Ala Thr
1               5
```

<210> 10
<211> 8
<212> PRT
<213> Homo sapiens

<400> 10

```
Leu Gln Ile Tyr Asn Met Pro Ile
1               5
```

<210> 11
<211> 126
<212> PRT
<213> Homo sapiens

<400> 11

```
Gln Val Glu Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
            35                  40                  45

Ser Ala Ile Asn Ala Ser Gly Thr Arg Thr Tyr Tyr Ala Asp Ser Val
            50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                    85                  90                  95
```

```
         Ala Arg Gly Lys Gly Asn Thr His Lys Pro Tyr Gly Tyr Val Arg Tyr
                     100                 105                 110

         Phe Asp Val Trp Gly Gln Gly Thr Leu Val Thr Val Ser Ser
                     115                 120                 125
```

<210> 12
<211> 110
<212> PRT
<213> Homo sapiens

<400> 12

```
         Asp Ile Val Leu Thr Gln Ser Pro Ala Thr Leu Ser Leu Ser Pro Gly
         1                   5                   10                  15

         Glu Arg Ala Thr Leu Ser Cys Arg Ala Ser Gln Ser Val Ser Ser Ser
                     20                  25                  30

         Tyr Leu Ala Trp Tyr Gln Gln Lys Pro Gly Gln Ala Pro Arg Leu Leu
                     35                  40                  45

         Ile Tyr Gly Ala Ser Ser Arg Ala Thr Gly Val Pro Ala Arg Phe Ser
                     50                  55                  60

         Gly Ser Gly Ser Gly Thr Asp Phe Thr Leu Thr Ile Ser Ser Leu Glu
         65                  70                  75                  80

         Pro Glu Asp Phe Ala Thr Tyr Tyr Cys Leu Gln Ile Tyr Asn Met Pro
                     85                  90                  95

         Ile Thr Phe Gly Gln Gly Thr Lys Val Glu Ile Lys Arg Thr
                     100                 105                 110
```

<210> 13
<211> 32
<212> PRT
<213> Artificial Sequence

<220>
<223> Peptide linker

<400> 13

```
         Gly Gly Gly Gly Ser Gly Gly Gly Gly Ser Gly Gly Gly Gly Ser Gly
         1                   5                   10                  15

         Gly Gly Gly Ser Gly Gly Gly Gly Ser Gly Gly Gly Gly Ser Gly Gly
                     20                  25                  30
```

<210> 14

<211> 15
<212> PRT
<213> Artificial Sequence

<220>
<223> Epitope mapping peptide

<400> 14

```
        Ile Gly Gln Asn Met Val Thr Ile Val Gln Ser Asn Gly Asn Leu
        1               5                   10                  15
```

<210> 15
<211> 15
<212> PRT
<213> Artificial Sequence

<220>
<223> Epitope mapping peptide

<400> 15

```
        Asn Met Val Thr Ile Val Gln Ser Asn Gly Asn Leu Asp Pro Val
        1               5                   10                  15
```

<210> 16
<211> 15
<212> PRT
<213> Artificial Sequence

<220>
<223> Epitope mapping peptide

<400> 16

```
        Gln Ser Asn Gly Asn Leu Asp Pro Val Glu Ser Pro Glu Gly Tyr
        1               5                   10                  15
```

<210> 17
<211> 20
<212> PRT
<213> Artificial Sequence

<220>
<223> Peptide linker

<400> 17

```
        Gly Gly Gly Gly Ser Gly Gly Gly Gly Ser Gly Gly Gly Gly Ser Gly
        1               5                   10                  15

        Gly Gly Gly Ser
                    20
```

**EP 2 890 712 B1**